(19) Europäisches Patentamt / European Patent Office / Office européen des brevets

(11) **EP 3 172 557 B1**

(12) **FASCICULE DE BREVET EUROPEEN**

(45) Date de publication et mention de la délivrance du brevet:
**28.11.2018 Bulletin 2018/48**

(21) Numéro de dépôt: **15739294.5**

(22) Date de dépôt: **21.07.2015**

(51) Int Cl.:
**G01N 23/04** *(2018.01)* **A61B 5/00** *(2006.01)*

(86) Numéro de dépôt international:
**PCT/EP2015/066619**

(87) Numéro de publication internationale:
**WO 2016/012435 (28.01.2016 Gazette 2016/04)**

(54) **SYSTEME D'IMAGERIE PAR RAYONS X PERMETTANT LA CORRECTION DE LA RADIATION DIFFUSEE ET LA DETECTION PRECISE DE LA DISTANCE SOURCE DETECTEUR**

RÖNTGENBILDGEBUNGSSYSTEM MIT FUNKTION ZUR KORREKTUR DER STREUUNGSSTRAHLUNG UND GENAUEN ERKENNUNG DES ABSTANDES ZWISCHEN QUELLE UND DETEKTOR

X-RAY IMAGING SYSTEM ALLOWING THE CORRECTION OF THE SCATTER RADIATION AND PRECISE DETECTION OF THE DISTANCE BETWEEN THE SOURCE AND THE DETECTOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.07.2014 FR 1457067**

(43) Date de publication de la demande:
**31.05.2017 Bulletin 2017/22**

(73) Titulaires:
- **Université Grenoble Alpes**
  **38400 Saint-Martin-d'Hères (FR)**
- **Surgiqual Institute**
  **38700 La Tronche (FR)**

(72) Inventeurs:
- **CINQUIN, Philippe**
  **F-38330 St Nazaire Les Eymes (FR)**
- **DESBAT, Laurent**
  **F-38700 La Tronche (FR)**
- **GRONDIN, Yannick**
  **73800 Arbin (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**US-A1- 2012 207 370**

- **SCHORNER K ET AL: "Scatter Correction Method by Temporal Primary Modulation in X-Ray CT", IEEE TRANSACTIONS ON NUCLEAR SCIENCE, IEEE SERVICE CENTER, NEW YORK, NY, US, vol. 59, no. 6, 1 décembre 2012 (2012-12-01), pages 3278-3285, XP011487530, ISSN: 0018-9499, DOI: 10.1109/TNS.2012.2218127**
- **DALY M ET AL: "Geometric calibration of a mobile C-arm for intraoperative cone-beam CT", MEDICAL PHYSICS, AIP, MELVILLE, NY, US, vol. 35, no. 5, 28 avril 2008 (2008-04-28), pages 2124-2136, XP012116074, ISSN: 0094-2405, DOI: 10.1118/1.2907563**
- **AHN SO HYUN ET AL: "Development of a beam stop array system with dual scan mode for scatter correction of cone-beam CT", JOURNAL OF THE KOREAN PHYSICAL SOCIETY, KOREAN PHYSICAL SOCIETY, KR, vol. 64, no. 8, 7 mai 2014 (2014-05-07), pages 1220-1229, XP035315286, ISSN: 0374-4884, DOI: 10.3938/JKPS.64.1220 [extrait le 2014-05-07]**

EP 3 172 557 B1

**Description**

## DOMAINE TECHNIQUE ET ART ANTÉRIEUR

La présente invention concerne le domaine de l'imagerie numérique par rayons X.

En imagerie par rayons X, on distingue dans le rayonnement détecté un rayonnement P direct appelé « primaire » et un rayonnement S appelé « secondaire » ou encore « diffusé » et qui provient principalement de la diffusion par effet Compton.

Dans le cas de la radiologie conventionnelle et interventionnelle, le rapport S/P peut être supérieur à 5, tandis qu'en mammographie, ce rapport est couramment situé entre 0.3 et 1.5.

Le contraste normalisé Cs d'une image en présence de rayonnement diffusé peut s'exprimer en fonction d'un contraste normalisé Cp d'une image constituée uniquement de rayonnement primaire P par la relation suivante :

$$C_s = \frac{Cp}{1+\frac{S}{P}}.$$

Ainsi, le rayonnement secondaire dégrade de manière significative le contraste de l'image radiologique.

On cherche donc généralement à réduire au maximum sa contribution.

Pour cela, une solution consiste à prévoir une grille anti-diffusante disposée entre un détecteur encore appelé imageur et un objet cible, par exemple un patient, dont on souhaite obtenir une radiographie.

Cette grille anti-diffusante permet de réduire le rapport S/P mais a pour inconvénient de diminuer par la même occasion la contribution du primaire P.

Pour atteindre un niveau de signal sur bruit dans l'image identique à celui d'un système sans grille anti-diffusante, il est alors généralement nécessaire d'augmenter le temps d'exposition ou l'intensité du rayonnement. Il en résulte une augmentation de la dose de rayonnement au patient d'un facteur qui peut être compris entre 2 et 8 par rapport à celle employée en radiologie conventionnelle sans utilisation de la grille.

Une manière alternative pour corriger la contribution du rayonnement secondaire consiste à effectuer un traitement numérique sur l'image obtenue.

Les documents FR 2 759 800 et US 7 551 716 donnent des exemples de méthodes de traitement numérique d'image radiographique.

Une autre manière de corriger l'effet d'un rayonnement diffus est d'employer un élément bloqueur configuré pour être déplacé entre une première position et une deuxième position, de sorte que dans la première position, une zone opaque occupe entièrement un premier emplacement donné et une zone transparente occupe entièrement un deuxième emplacement donné, dans la deuxième position, le premier emplacement donné est entièrement occupé par une zone transparente, le deuxième emplacement donné étant entièrement occupé par une zone opaque. Un tel élément est, par exemple, décrit dans Schörner et al., "Scatter correction method by temporal primary modulation in X-ray CT", IEEE Transactions on Nuclear Science, vol. 59 (2012) 3278-3285.

Des méthodes de traitement d'image utilisant un modèle 3D préenregistré de l'objet cible sont efficaces pour des applications, telles que la mammographie, où la forme varie relativement peu d'un objet cible à l'autre. Pour d'autres applications telles que la radiologie conventionnelle, ces méthodes sont plus difficiles à mettre en oeuvre et sont susceptibles d'impliquer une lourdeur de calcul qui conduit à un temps de traitement important.

En plus des artéfacts dus au rayonnement diffusé, les images obtenues par un système d'imagerie par rayons X peuvent souffrir d'un deuxième type d'artéfacts dus à une variation de la position du détecteur par rapport à la source.

De tels artéfacts apparaissent en particulier dans les systèmes d'imagerie 3D tel que les systèmes de type à arceau (« C-arm selon la terminologie anglo-saxonne »).

Il peut donc s'avérer important de pouvoir effectuer un étalonnage de ces systèmes encore appelé calibration afin de pouvoir corriger ce deuxième type d'artéfacts.

Des méthodes de calibration utilisant des capteurs inertiels afin de suivre le mouvement réel des différents composants du système d'imagerie sont connus.

Leur inconvénient réside principalement dans le manque de fiabilité des capteurs qui peuvent être sujets à des perturbations.

Une méthode de calibration alternative utilisant des marqueurs radio-opaques est décrite par exemple dans le document US 6 490 475.

Avec une telle méthode, l'image des marqueurs vient interférer avec l'image de l'objet dont on souhaite acquérir l'image.

Le document « On-line C-arm Intrinsic Calibration: Simulation Study », de B. Spencer & L. Desbat, IEEE Medical imaging conférence 2014, présente une autre méthode de calibration à l'aide cette fois de 4 marqueurs opaques liés à la source.

Le document US7497621 propose quant à lui une méthode de calibration à l'aide d'une grille radio-transparente. Une telle méthode peut, dans certains cas, manquer de précision dans la mesure où elle nécessite de détecter un motif correspondant à un objet-radio transparent dans une image qui peut manquer de contraste.

Il se pose le problème de trouver un nouveau procédé d'imagerie par rayons X pour l'acquisition d'image(s), et un nouveau système d'imagerie planaire par rayons X amélioré vis à vis d'inconvénients mentionnés ci-dessus.

En particulier, il se pose le problème de mettre en oeuvre un dispositif qui puisse permettre à la fois de pouvoir réaliser un étalonnage précis d'un système d'imagerie par rayonnement X ainsi qu'une correction du rayonnement diffusé dans une image radiographique.

## EXPOSÉ DE L'INVENTION

Un mode de réalisation de l'invention prévoit un système d'imagerie par rayons X muni d'un dispositif comprenant :

- un élément bloqueur destiné à être disposé entre une source de rayons X et un objet cible dont on souhaite obtenir une image, l'élément bloqueur étant formé d'une alternance d'une ou plusieurs zones opaques permettant le blocage de rayons X et d'une ou plusieurs zones transparentes permettant le passage des rayons X,
- des moyens de déplacement de l'élément bloqueur configurés pour déplacer l'élément bloqueur entre au moins une première position et au moins une deuxième position,

le dispositif étant configuré de sorte que :

- dans la première position, une zone opaque occupe entièrement un premier emplacement donné et une zone transparente occupe entièrement un deuxième emplacement donné,
- dans la deuxième position, le premier emplacement donné est entièrement occupé par une zone transparente, le deuxième emplacement étant entièrement occupé par une zone opaque.

Le système d'imagerie planaire par rayons X comprend en outre :

- une source de rayons X,
- un détecteur de rayons X,
- des moyens d'acquisition d'image configurés pour réaliser une première acquisition d'une première trame d'image ou première image lorsque l'élément bloqueur se trouve dans la première position et pour réaliser une deuxième acquisition d'une deuxième trame d'image ou deuxième image lorsque l'élément bloqueur se trouve dans la deuxième position.

Le système d'imagerie par rayons X comprend en outre une unité de traitement d'image configurée pour déterminer une position du détecteur à partir de coordonnées d'un ou plusieurs motifs projetés du bloqueur dans la première image et/ou dans la deuxième image.

Avec un tel bloqueur, on obtient des images contrastées et riches en information ce qui permet de déterminer précisément la position du détecteur.

Cette détermination est importante à la fois pour permettre de calibrer le système d'imagerie et pouvoir effectuer une correction de la contribution du rayonnement diffusé dans une image.

Selon une possibilité de mise en oeuvre, l'unité de traitement d'image comporte ou est couplée à une mémoire pour stocker des paramètres d'une première matrice de transformation géométrique reliant des coordonnées de motifs de référence avec respectivement les coordonnées d'éléments du bloqueur, chaque motif de référence correspondant à une projection d'un élément du bloqueur dans une image radiographique dite « de référence » générée lorsque le détecteur est situé à une distance de référence de la source tandis que le bloqueur se trouve dans une position donnée parmi lesdites première et deuxième positions.

La position de référence du détecteur correspond de préférence à la position qui est utilisée par l'algorithme de reconstruction d'image. Autrement dit la position de référence constitue la position nominale pour effectuer la reconstruction d'image.

Une image de référence est une image comportant des motifs du bloqueur et qui est acquise sans objet cible ou objet intermédiaire entre le bloqueur et le détecteur.

L'unité de traitement d'image peut être alors en outre configurée pour :

- identifier des motif projetés d'éléments du bloqueur dans une autre image radiographique dite « d'alignement » générée lorsque le bloqueur se trouve dans ladite position donnée, et pour apparier les motifs projetés dans l'image d'alignement avec respectivement des éléments du bloqueur,
- pour calculer des paramètres d'une deuxième matrice de transformation géométrique reliant les coordonnées des motifs projetés dans l'image radiographique d'alignement avec les coordonnées des motifs de référence, et pour calculer la position du détecteur à partir des paramètres des première et deuxième matrices.

Une image d'alignement peut être acquise cette fois lorsqu'un objet cible se trouve entre le bloqueur et le détecteur. Cet objet cible peut être par exemple un patient lorsque le système d'imagerie est dédié à l'imagerie médicale.

Un mode de réalisation de la présente invention prévoit également un procédé de calibration d'un système d'imagerie radiographique tel que défini plus haut et comprenant les étapes suivantes :

- disposer le bloqueur entre la source et le détecteur,
- faire l'acquisition par le détecteur d'au moins une image radiographique dite d'alignement comportant un ou plusieurs motifs projetés du bloqueur ;
- déterminer des coordonnées de motifs projetés du bloqueur dans l'image d'alignement et
- calculer une position du détecteur à partir de coordonnées des motifs projetés dans l'image radiographique d'alignement.

Le procédé peut comprendre en outre une étape consistant à former, à l'aide de moyens de traitement informatiques, une image corrigée de l'objet cible.

Pour cela, on peut estimer le rayonnement primaire d'au moins un pixel donné du détecteur en fonction de son appartenance à une première catégorie de pixels ou à une deuxième catégorie de pixels, l'appartenance à la première ou à la deuxième catégorie dépendant d'un critère $q_1$-$q_2$ prédéterminé propre à chaque pixel et qui peut avoir été estimé préalablement, avec $q_1$, $q_2$ des paramètres prédéterminés représentatifs de fractions de rayonnement primaire reçus par le pixel donné du détecteur respectivement dans la première position du bloqueur et dans la deuxième position du bloqueur.

On peut alors distinguer une première catégorie de pixels pour lesquels le critère $q_1$-$q_2$ est différent de 0 ou supérieur à un seuil z donné.

Pour un pixel n donné appartenant à cette première catégorie, l'estimation $\hat{P}$ du rayonnement primaire reçu par le pixel donné n, peut dépendre d'un rapport entre :

- une différence $I_1(n)$-$I_2(n)$ entre une intensité $I_1(n)$ de rayonnement primaire détectée par le pixel donné n lors de la première acquisition et d'une intensité $I_2(n)$ de rayonnement primaire détectée par le pixel n lors de la deuxième acquisition,
- une différence $q_1(n)$-$q_2(n)$.

On peut distinguer une deuxième catégorie de pixels pour lesquels le critère $q_1$-$q_2$ est égale 0 ou inférieure à un seuil z donné.

Dans ce cas, pour un pixel m appartenant à la deuxième catégorie, l'estimation $\hat{P}$ (m) du rayonnement primaire reçu par le pixel m, peut être fonction d'une intensité $I_1(m)$ de rayonnement primaire détectée par le pixel m lors de la première acquisition d'une intensité $I_2(m)$ du rayonnement primaire détectée par le pixel m lors de la deuxième acquisition, et de $\hat{S}$ int(n) une valeur estimée par interpolation du rayonnement diffusé du pixel m à partir d'une valeur de rayonnement diffusé calculé pour ses pixels voisins.

Le critère $q_1$-$q_2$ peut être déterminé préalablement à l'acquisition des trames d'images, en effectuant une calibration durant laquelle une première acquisition d'une première trame d'image puis une deuxième acquisition d'une deuxième trame d'image, le tout dans un dispositif sans objet cible, sont effectuées.

En vue de constituer l'image de l'objet cible, on peut déduire le critère $q_1$-$q_2$ pour un pixel donné en fonction de la localisation de ce pixel donné par rapport au bloqueur dans la première position et dans la deuxième position.

Pour effectuer cette localisation on peut estimer le positionnement du bloqueur relativement au détecteur en analysant une des trames d'image, par exemple la première trame. La connaissance précise du positionnement du détecteur peut donc également être utilisée afin de corriger la contribution du rayonnement diffusé.

Pour un pixel donné, une valeur de $q_1$-$q_2$ peut être extraite d'un fichier de calibration pré-enregistré ou estimée par calcul.

Selon un exemple n'étant pas couvert par la présente invention, dans un cas où le système est un système d'imagerie planaire adapté pour réaliser une acquisition d'image(s) en deux dimensions, le système peut être configuré de sorte que la source, l'élément bloqueur, et l'imageur ont lors de la première acquisition un premier agencement et lors de la deuxième acquisition un deuxième agencement différent de premier agencement, le positionnement de la source et du détecteur par rapport à l'objet cible ou à un support sur lequel repose l'objet cible, étant le même dans le premier agencement et le deuxième agencement.

Ainsi cet exemple prévoit également un procédé d'imagerie, par rayons X, pour l'acquisition d'image(s) en 2 dimensions.

Ce procédé comprend des étapes consistant à :

- réaliser une première acquisition d'une première trame d'image d'un objet cible à l'aide d'un détecteur doté d'une pluralité de pixels, par exposition de l'objet cible à une source de rayons X, un élément bloqueur étant disposé entre la source et l'objet cible, l'élément bloqueur étant formé d'une alternance d'une ou plusieurs zones opaques permettant le blocage de rayons X et d'une ou plusieurs zones transparentes permettant le passage des rayons X, la source, l'élément bloqueur, et le détecteur ayant lors de la première acquisition un premier agencement dans lequel l'élément bloqueur a une première position par rapport au détecteur,
- déplacer l'élément bloqueur par rapport au détecteur, de sorte que l'élément bloqueur passe de la première position à une deuxième position par rapport au détecteur, puis,
- réaliser une deuxième acquisition d'une deuxième trame d'image de l'objet cible par exposition de l'objet cible à la source de rayons X, la source, l'élément bloqueur, et le détecteur ayant lors de la deuxième acquisition un deuxième agencement différent du premier agencement et dans lequel l'élément bloqueur se trouve dans la deuxième position.

Le positionnement de la source et du détecteur par rapport à l'objet cible ou à un support sur lequel repose l'objet cible, peut être quant à lui le même et conservé entre le premier agencement et le deuxième agencement.

Dans un tel système non couvert par la présente invention, l'élément bloqueur est susceptible d'adopter une première position et une deuxième position permettant l'acquisition de trames d'images complémentaires.

Pour cela, la première position et la deuxième position de l'élément bloqueur peuvent être prévues de sorte que :

- dans la première position, une zone opaque de l'élément bloqueur occupe un premier emplacement dans un périmètre donné destiné à être traversé par un faisceau de rayons X issu de la source et une zone transparente de l'élément bloqueur occupe un deuxième emplacement dans le périmètre donné,
- dans la deuxième position, le premier emplacement et le deuxième emplacement sont occupés respectivement par une zone transparente et une zone opaque de l'élément bloqueur.

Selon une possibilité de mise en oeuvre, l'élément bloqueur, la première position du bloqueur et la deuxième position du bloqueur peuvent être prévues de sorte que : dans la première position, une zone opaque de l'élément bloqueur occupe un premier emplacement symétrique de celui occupé par une autre zone opaque de l'élément bloqueur dans la deuxième position, par rapport à un plan passant par la source et qui est orthogonal à une face avant du bloqueur.

Selon une possibilité de mise en oeuvre, la première position et la deuxième position de l'élément bloqueur sont prévues de sorte que :

- dans la première position au moins un premier pixel du détecteur est disposé dans l'ombre ou la pénombre de l'élément bloqueur tandis qu'au moins un deuxième pixel est directement éclairé par la source de rayon X,
- dans la deuxième position, le deuxième pixel se trouve dans l'ombre de l'élément bloqueur tandis que le premier pixel est directement éclairé lorsque dans la première position il se trouve dans l'ombre ou bien se trouve dans la pénombre de l'élément bloqueur lorsque dans la première position il est dans la pénombre de l'élément bloqueur.

Dans ce cas, un troisième pixel disposé sur le détecteur de sorte que le deuxième pixel se trouve entre le premier pixel et ce troisième pixel, peut se trouver :

- dans l'ombre de l'élément bloqueur lorsque l'élément bloqueur est dans la première position,
- directement éclairé par la source de rayon X lorsque l'élément bloqueur est dans la deuxième position.

Selon une première possibilité de réalisation, l'acquisition de la première trame d'image peut être effectuée par exposition à une première impulsion de rayonnement X émise par la source, tandis que la deuxième acquisition est réalisée par une deuxième exposition à une deuxième impulsion de rayonnement X émise par la source de rayons X, la source de rayons X étant éteinte ou n'émettant pas de rayonnement entre la première et la deuxième impulsion.

Selon une deuxième possibilité de mise en oeuvre, l'acquisition de la première trame d'image est réalisée par exposition de l'objet cible à une impulsion de rayonnement X émise par la source, tandis que l'acquisition de la deuxième trame d'image est réalisée par exposition de l'objet cible à cette même impulsion de rayonnement X, l'élément bloqueur étant alors mobile pendant la durée de cette impulsion.

Selon une possibilité de mise en oeuvre, le déplacement de l'élément bloqueur entre la première position et la deuxième position se fait par translation de l'élément bloqueur d'un pas p donné et dans un plan parallèle au détecteur.

Ce pas p de translation peut être égal à k*l, avec k un nombre entier, l étant la largeur de zones opaques de l'élément bloqueur.

Selon une possibilité de mise en oeuvre de l'élément bloqueur, celui-ci peut être doté d'une face avant comportant une alternance de zones opaques et de zones transparentes parallèles entre elles.

En variante, un élément bloqueur dont la face avant comporte des zones opaques et des zones transparentes ayant une disposition en damier peut être prévue.

Selon une autre variante de mise en oeuvre, le déplacement de l'élément bloqueur entre la première et la deuxième position peut être réalisé par rotation de l'élément bloqueur autour d'un axe passant par la source de rayons X.

Dans ce cas, l'élément bloqueur peut avoir un profil incurvé.

Dans une variante, le bloqueur est constitué de zones opaques et transparentes formant un arc de cercle dont le centre est la source de rayons X .

BRÈVE DESCRIPTION DES DESSINS

La présente invention sera mieux comprise à la lecture de la description d'exemples de réalisation donnés, à titre purement indicatif et nullement limitatif, en faisant référence aux dessins annexés sur lesquels :

la figure 1 illustre un exemple de système d'imagerie planaire par rayons X muni d'un élément bloqueur de rayons X déplaçable entre plusieurs positions d'acquisitions de trames d'image ;

la figure 2 illustre un exemple particulier d'agencement de zones transparentes et de zones opaques au niveau d'une face avant d'un élément bloqueur de rayons X ;

la figure 3 illustre un exemple de dispositif configuré pour déplacer un élément bloqueur de rayons X ;

les figures 4A-4B et 5A-5B et 6 illustrent différentes positions d'un bloqueur dans un système d'imagerie planaire, le passage d'une position à l'autre du bloqueur étant effectué dans cet exemple par translation ;

la figure 7 illustre un exemple d'éclairement des pixels d'un détecteur dans un système d'imagerie muni d'un bloqueur ;

la figure 8 illustre un exemple de module de traitement informatique d'image pour constituer une image à partir de trames d'images complémentaires ou d'images complémentaires obtenues dans des positions complémentaires d'un élément bloqueur ;

la figure 9 illustre un exemple d'organigramme de traitement effectué par un module de traitement informatique pour constituer une image à partir de trames d'images complémentaires obtenues dans des positions complémentaires d'un élément bloqueur ;

la figure 10 illustre un autre exemple particulier d'agencement de zones transparentes et de zones opaques au niveau d'une face avant d'un élément bloqueur de rayons X ;

la figure 11 illustre un exemple de dispositif dans lequel le bloqueur comporte des zones transparentes et des zones opaques réparties sur une portion courbe ;

la figure 12 illustre un exemple de système d'imagerie planaire par rayons X de type à bras en C (« C-arm), muni d'un élément bloqueur et dans lequel le module de traitement d'image est configuré pour déterminer une position entre la source et le détecteur au moyen de l'analyse d'au moins une image du bloqueur ;

la figure 13 illustre un exemple de modélisation de repères mise en oeuvre par le module de traitement d'image pour déterminer une position entre la source et le détecteur ;

Des parties identiques, similaires ou équivalentes des différentes figures portent les mêmes références numériques de façon à faciliter le passage d'une figure à l'autre.

Les différentes parties représentées sur les figures ne le sont pas nécessairement selon une échelle uniforme, pour rendre les figures plus lisibles.

## EXPOSÉ DÉTAILLÉ DE MODES DE RÉALISATION PARTICULIER

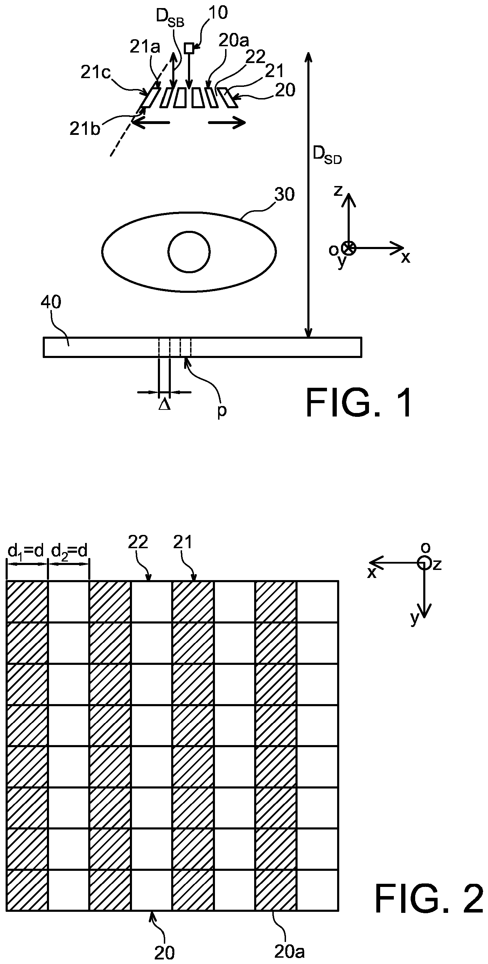

Un exemple de réalisation d'un système d'imagerie planaire par rayons X est illustré sur la figure 1.

Un tel système peut être appliqué par exemple à la mammographie, ou à la radiologie conventionnelle et/ou interventionnelle (fluoroscopie) et permet l'acquisition de plusieurs trames d'images afin de former une image d'un objet cible 30 ou d'une région de cet objet cible 30, l'objet cible 30 étant par exemple un patient, éventuellement placé sur un support (non représenté).

Les trames d'image de l'objet cible 30 sont produites à l'aide d'un détecteur 40 numérique, formé d'une pluralité d'éléments de détection encore appelés « pixels », qui sont agencés selon une matrice.

Afin de corriger le rayonnement diffusé sur l'image de l'objet cible 30, le système comporte un élément bloqueur 20 agencé entre une source 10 de rayons X et l'objet cible 30, ce dernier étant lui-même placé entre l'élément bloqueur 20 et le détecteur 40. Une trame d'image est une image obtenue dans une position donnée du bloqueur.

L'élément bloqueur 20 est formé d'une alternance de zones 21 opaques aux rayons X, c'est-à-dire qui ne laissent pas ou peu passer les rayons X, et de zones 22 transparentes aux rayons X. Les zones 21 opaques sont à base d'un matériau ayant un pouvoir important d'absorption aux rayons X tel que par exemple le plomb ou le tungstène.

A titre indicatif, aux énergies utilisées en radiologie conventionnelle, des zones d'épaisseur de plusieurs millimètres à base de plomb sont opaques vis-à-vis des rayons X. A 60 keV par exemple, le facteur de transmission de zones de plomb de 3 mm est par exemple de l'ordre de $2*10^{-7}$.

Les zones transparentes 22 peuvent être sous forme de fentes vides ou à base d'un matériau très peu absorbant vis-à-vis des rayons X, par exemple tel que du carbone.

Les zones opaques 21 peuvent être sous forme de blocs parallélépipédiques comportant des faces latérales 21c reliant une face avant 21a disposée en regard de la source de rayons X, une face arrière 21b disposée en regard de l'objet cible et du détecteur 40, les faces latérales 21c étant inclinées les unes par rapport aux autres et orientées selon des directions respectives différentes focalisées vers la source 10 autrement dit qui convergent vers la source de rayons X.

Dans l'exemple de réalisation illustré sur la figure 1, le dispositif montré selon une vue de dessus, comporte un bloqueur 20 de forme trapézoïdale, ayant une face avant 20a disposée en regard de la source de rayons X, cette face avant 20a étant de section plus faible que celle de sa face arrière 20b opposée à la face avant 20a et disposée en regard de l'objet cible 30 et du détecteur 40.

Un exemple particulier d'agencement de la face avant 20a du bloqueur 20 est illustré sur la figure 2. Les zones transparentes 22 ont dans cet exemple une forme rectangulaire semblable à celle des zones opaques 21, les zones transparentes 22 ayant des largeurs (mesurées dans une direction parallèle au plan [0 ; x ; y] du repère orthogonal [0 ; x; y; z]) égales à d, avec d qui peut être comprise par exemple entre 0.2 et 5 mm. La valeur de 0.2 mm peut correspondre à l'ordre de grandeur de la taille $\Delta$=0.2 mm d'un pixel, la valeur de 5 mm correspondant à plusieurs dizaines de fois la taille d'un pixel.

Un bloqueur 20 avec des zones opaques de largeur d faible permet une meilleure estimation du rayonnement diffusé tandis qu'un bloqueur doté de zones opaques de largeur d plus importante permet de réduire la part de pénombre par rapport à la part d'ombre dans la trame d'image acquise. On peut donc prévoir un bloqueur en réalisant un compromis entre ces deux critères.

Avec un tel système imageur, on réalise une acquisition de plusieurs trames d'image, chacune de ces trames d'image pouvant être obtenue dans une position différente de l'élément bloqueur 20.

L'élément bloqueur 20 est ainsi amené à être déplacé entre plusieurs positions, ce déplacement étant, selon l'exemple particulier de réalisation illustré sur la figure 1, une translation, de préférence dans un plan parallèle au plan principal du détecteur (i.e. un plan passant par le détecteur et parallèle au plan [0 ; x ; z] sur le dispositif de la figure 1).

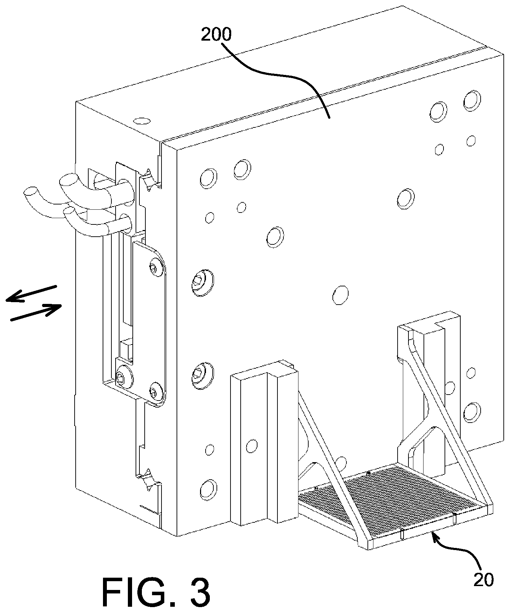

La translation peut être effectuée par le biais d'un dispositif de déplacement motorisé, par exemple une table 200 de positionnement linéaire mono-axe telle que celle illustrée par exemple sur la figure 3, et sur laquelle le bloqueur 20 est fixé.

L'ensemble table de positionnement 200 et bloqueur 20 peut être monté avec la source 10 et intégré sur le tube radiologique (non représenté).

Le déplacement en translation de l'élément bloqueur 20 se fait entre deux acquisitions de trames d'images successives, selon un pas p donné dans une direction donnée appartenant à un plan parallèle au plan principal du détecteur 40, avec p le pas de translation qui peut être égal à k*d, k étant un nombre entier non-nul, d étant la largeur des zones opaques 21 du bloqueur vue de la source 10.

Pendant l'acquisition des trames d'image et entre deux acquisitions successives, le positionnement de la source 10 et du détecteur 40 par rapport à l'objet cible 30 ou à un support sur lequel repose l'objet cible 30, reste le même. Ainsi, de préférence, seul l'élément bloqueur 20 se déplace pour effectuer les différentes acquisitions de trames d'image.

La face avant 20a de l'élément bloqueur 20 peut être située à une distance DSB par exemple comprise entre 5 cm et 20 cm de la source 10 de rayons X. La distance DSD entre la source 10 de rayons X et le détecteur 40 peut être par exemple comprise entre 70 cm et 120 cm. Ces valeurs sont données à titre d'exemple pour réaliser une radiographie.

La tension appliquée aux électrodes du tube de rayons X peut être semblable à celle d'un système classique, par exemple de l'ordre de plusieurs dizaines de kV. Pour chaque trame d'image, la fluence énergétique délivrée par la source 10 peut être égale à celle d'un système d'imagerie classique et n'utilisant pas de bloqueur ou de grille anti-diffusante.

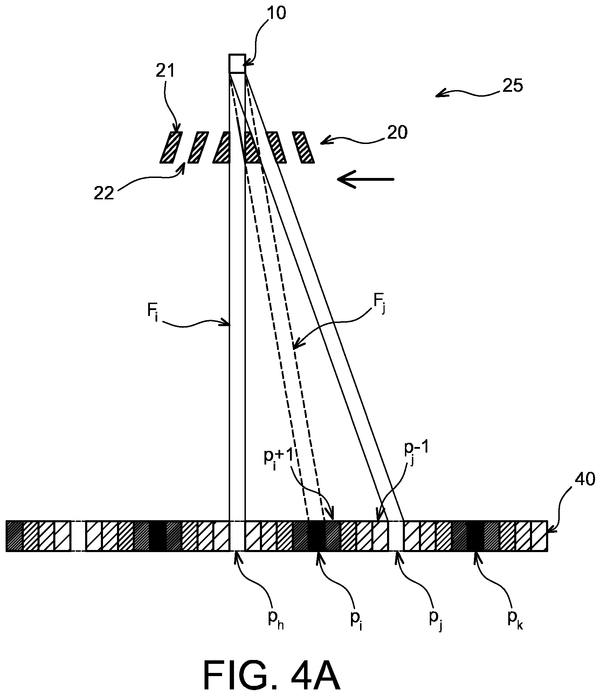

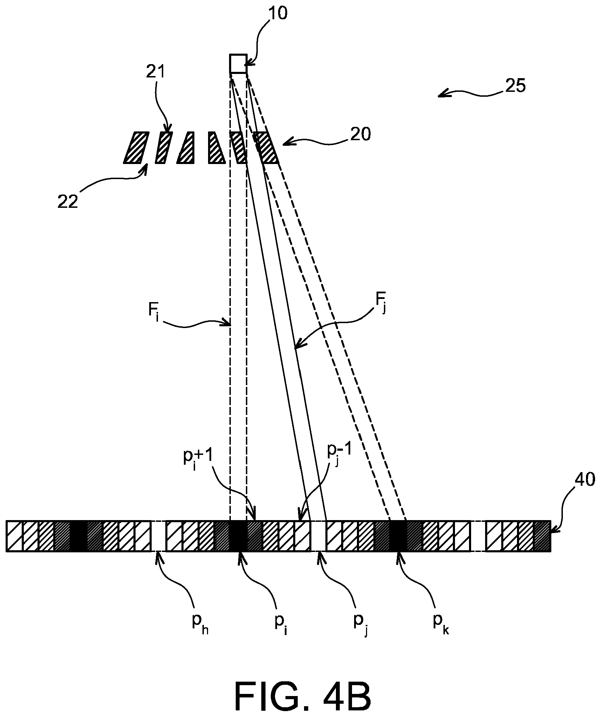

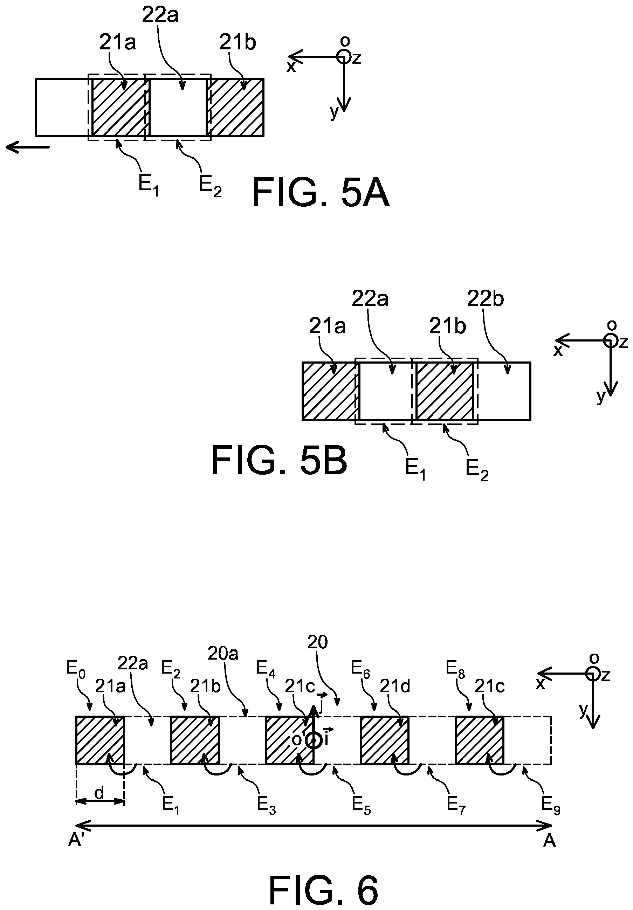

Selon un mode de réalisation illustré sur les figures 4A-4B et 5A-5B (les figures 5A-5B illustrant des positions de la face avant bloqueur vues de la source de rayons X), on peut prévoir l'acquisition de deux trames d'image complémentaires avec deux positions différentes du bloqueur 20, une première acquisition d'une première trame d'image étant effectuée dans une première position du bloqueur 20 par rapport au détecteur 40 (figures 4A et 5A), une deuxième acquisition d'une deuxième trame d'image étant effectuée dans une deuxième position du bloqueur 20 par rapport au détecteur 40 (figures 4B et 5B).

Le positionnement de la source 10 et du détecteur 40 par rapport à l'objet cible 30 ou à un support (non représenté) sur lequel se situe l'objet cible, ne varie pas entre la première acquisition et la deuxième acquisition. De préférence, la source 10 et le détecteur 40 sont immobiles entre les deux acquisition de trame d'image. De préférence également, l'objet cible 30, par exemple un patient, est également immobile entre la première et la deuxième acquisition.

Le positionnement des zones opaques 22 du bloqueur 20 dans la première position du bloqueur est de préférence prévu complémentaire de celle des zones opaques 22 du bloqueur 20 lorsqu'il se trouve dans la deuxième position.

Ainsi, dans la première position du bloqueur 20, une zone opaque 21a du bloqueur 20 occupe un premier emplacement E1 dans un périmètre donné (périmètre 25 sur les figures 4A et 4B) traversé par le faisceau de rayons X issu de la source 10 tandis qu'une zone transparente 22a du bloqueur 20 occupe un deuxième emplacement E2 dans le périmètre donné (figure 5A).

Dans la deuxième position, le premier emplacement E1 et le deuxième emplacement E2 sont occupés respectivement par la zone transparente 22a et une zone opaque 21b de l'élément bloqueur 20 (figure 5B).

Selon une possibilité de mise en oeuvre particulière illustrée sur la figure 6, le bloqueur 20 et ses déplacement peuvent être avantageusement prévus de telle manière que le positionnement de zones opaques dans la première position du bloqueur 20 comporte une symétrie avec le positionnement de zones opaques dans la deuxième position du bloqueur 20.

Dans l'exemple de la figure 6, la face avant 20a du bloqueur 20 vue depuis la source de rayons X est montrée dans la deuxième position du bloqueur 20, i.e. après déplacement en translation selon un pas ici par exemple égal à la largeur d d'une zone opaque. Sur cette figure, des zones $E_1$, $E_3$, $E_5$, $E_7$, $E_9$ en traits discontinus désignent des emplacements occupées auparavant par les zones opaques 21a, 21b, 21c, 21d, 21e dans la première position du bloqueur 20.

Ainsi, des emplacements $E_0$, $E_2$, $E_4$, $E_6$, $E_8$ occupés par les zones opaques 21a, 21b, 21c, 21d, 21e du bloqueur 20 dans la deuxième position, sont symétriques respectivement d'emplacements $E_9$, $E_7$, $E_5$, $E_3$, $E_1$ occupés par les zones opaques 21e, 21d, 21c, 21b, 21a dans la première position, si l'on considère un plan de symétrie [0' ; i ; j] (donné sur la figure 6 et qui est parallèle au plan [0 ; y ; z] du repère orthogonal [0 ; x ; y ; z]) orthogonal à la face avant 20a du bloqueur 20 et à un axe A'A de déplacement du bloqueur (l'axe de déplacement, étant parallèle au vecteur x du repère orthogonal [0 ; x ; y ; z) et passant par la source 10 et le centre de la face avant 20a bloqueur 20 entre une zone opaque et une zone transparente.

De ces différentes positions de l'élément bloqueur 20, il en résulte que dans la première position du bloqueur 20, des pixels pi, pk sont directement éclairés par la source 10 de rayon X tandis que d'autres pixels ph, pj, qui ne sont pas voisins des pixels pi, pk sont disposés dans l'ombre de l'élément bloqueur 20, le détecteur 40 comportant une alternance de pixels directement éclairés et de pixels dans l'ombre avec entre un pixel pi éclairé et un pixel pj dans l'ombre, des pixels $p_{i+1}$,..., $p_{j-1}$, qui se trouvent dans différents niveaux de pénombre et donc d'intensité (figure 4A).

Le déplacement de l'élément bloqueur 20 entre la première position et la deuxième position est prévu de sorte que dans la deuxième position (figure 4B), les pixels pi, pk se trouvent à présent dans l'ombre de l'élément bloqueur 20, tandis que les autres pixels ph, pj sont directement éclairés par la source de rayons X. Le détecteur 40 comporte alors également une alternance de pixels directement éclairés et de pixels dans l'ombre avec entre un pixel pj éclairé et un pixel pi dans l'ombre, des pixels $p_{i+1}$,..., $p_{j-1}$, qui se trouvent dans différents niveaux de pénombre et donc d'intensité. On pourra effectuer un traitement particulier des pixels qui parmi les pixels $p_{i+1}$,..., $p_{j-1}$, se trouvant dans la pénombre dans les deux positions, ont une intensité (ou niveau de pénombre ou niveau de gris) égal ou sensiblement égal ou sensiblement égale entre la première et la deuxième position.

Par pixel « directement éclairé », on entend ici qu'une partie du faisceau de rayonnement X Fi se propageant selon une direction rectiligne depuis la source 10 vers le détecteur 40 et atteignant ce pixel pi, ne rencontre pas de zone opaque 21 de l'élément bloqueur 20.

Par « pénombre ou ombre », on entend qu'une partie du faisceau de rayonnement X Fj se propageant selon une direction rectiligne depuis la source 10 vers le détecteur 40 et dirigé vers un pixel pj se trouvant dans l'ombre ou la pénombre, rencontre une zone opaque 21 de l'élément bloqueur 20, l'ombre étant un cas particulier de pénombre, pour lequel le pixel pj est totalement masqué par une zone opaque 21 du bloqueur et/ou reçoit une quantité nulle ou que l'on considère négligeable de photons. En présence d'un objet cible, les pixels dans l'ombre reçoivent majoritairement des rayons diffusés.

Les figures 4A-4B illustrent un cas, par exemple où l'image est une image numérique en niveaux de gris, la valeur de chaque pixel comportant une information d'intensité variant de noir, par exemple avec la valeur 0, traduisant la situation d'un pixel dans l'ombre, à blanc, par exemple avec valeur de 255, traduisant la situation d'un pixel directement éclairé, les pixels dans la pénombre étant représentés par différentes teintes de gris.

Par pixels pi et pj « qui ne sont pas voisins », on entend ici que les pixels pi, pj sont séparés par l'intermédiaire d'un ou plusieurs pixels $p_{i+1}$, ..., $p_{j-1}$ du détecteur 40.

Du fait de la complémentarité des deux trames d'images, le nombre total de pixels qui se trouvent directement éclairés pour l'ensemble des deux trames d'images peut être égal au nombre total de pixels qui se trouvent dans l'ombre lorsqu'on prend en compte l'ensemble des deux trames d'images.

Les figures 4A-4B sont données à titre d'exemple illustratif avec une taille donnée de pixel dans lequel des zones directement éclairées du détecteur et des zones du détecteur qui se trouvent dans l'ombre sont des pixels individuels $p_h$, $p_i$, $p_j$, $p_k$.

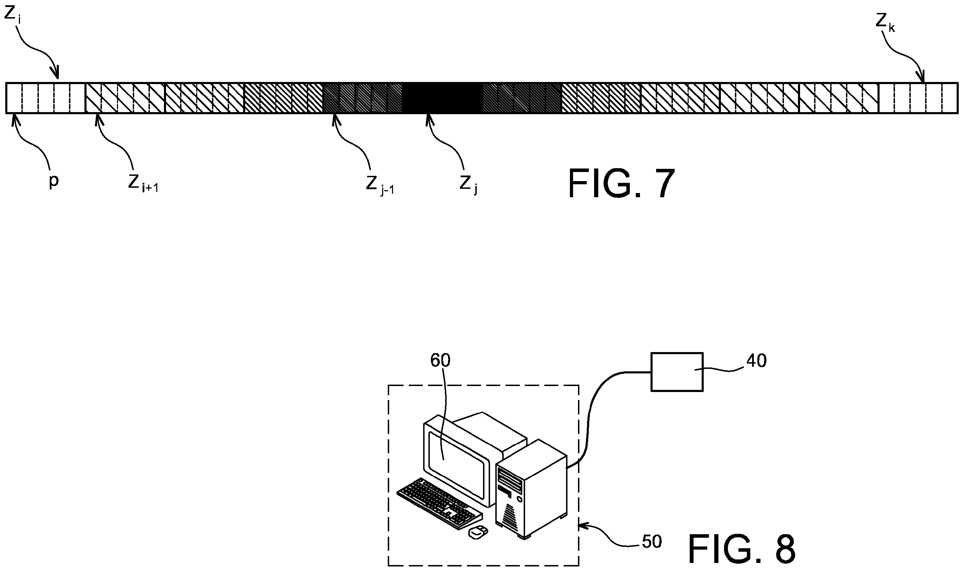

Toutefois, les zones directement éclairées $Z_i$, $Z_k$ du détecteur, les zones Zj du détecteur qui sont dans l'ombre et les zones $Z_{i+1}$,..., $Z_{j-1}$ peuvent être comme cela est illustré en variante sur la figure 7, des groupes de pixels p voisins.

On peut faire l'acquisition de la première trame d'image et de la deuxième trame d'image, complémentaire de la première trame, lors d'une exposition du détecteur 40 à une impulsion de rayonnement X émise par la source 10 durant laquelle l'élément bloqueur 20 se déplace.

En variante, on réalise une première exposition du détecteur 40 à une première impulsion de rayonnement X émise par la source 10 puis une deuxième exposition à une deuxième impulsion de rayonnement X émise par la source 10, la source de rayons X étant éteinte ou n'émettant pas de rayonnement entre la première et la deuxième impulsion.

Les deux positions de l'élément bloqueur 20 étant complémentaires pour réaliser l'acquisition des deux trames, le détecteur 40 peut être amené à recevoir une dose totale équivalente à celle d'une acquisition sans élément bloqueur. Le rapport signal à bruit sur l'image brute obtenue peut être égal à celui d'une image obtenue par le biais d'un système sans bloqueur.

Outre l'acquisition des trames d'images, on peut, préalablement à la constitution d'une image à partir de ces trames, avoir déterminé la position de l'élément bloqueur 20 relativement au détecteur 40 et ses pixels.

Cette détermination peut être réalisée après l'acquisition de la première trame d'image et/ou de la deuxième trame d'image, et peut être effectuée en analysant les trames d'images elles-mêmes.

Pour cela, l'analyse par un module informatique 50 ou une unité 50 de traitement de la trame d'image peut comporter une première étape consistant à détecter les motifs de la projection des éléments (zones opaques et/ou transparentes) du bloqueur sur l'image caractérisée par une alternance de zones (pixels) éclairées (intensité de signal fort) et de zones non éclairées (intensité de signal faible). Les bordures de ces zones qui sont des lignes peuvent être identifiées précisément par des méthodes de calcul utilisant la transformée de Fourier ou la transformée de Hough, telles que celles présentées dans R. Szeliski, Computer Vision : algorithms and applications, Springer 2010.

A partir de la détection dans l'image des motifs des zones opaques du bloqueur, il est ensuite possible en appliquant une méthode telle que celles développées pour la calibration de caméra dans le domaine de la vision par ordinateur, par exemple une méthode telle que décrite dans R. I. Hartley and A. Zisserman, Multiple View Geometry in Computer Vision, Cambridge University Press 2000, de déterminer les positions relatives du bloqueur et du détecteur.

Le choix de la méthode de localisation de l'élément bloqueur 20 par rapport à celle du détecteur 40 dépend de la géométrie de ce dernier.

Un traitement numérique est ensuite réalisé pour constituer l'image à partir des deux trames complémentaires.

Dans ce traitement, on peut considérer :

- l'intensité $I_1$ du rayonnement lu dans un pixel n donné lors de l'acquisition de la première trame d'image telle que $I_1(n) = q_1(n)P_1(n) + S_1(n)$, avec $q_1$ un facteur prédéterminé propre au pixel n, $P_1$ l'intensité de rayonnement primaire reçue par le pixel donné n lors de la première acquisition, $S_i$ l'intensité de rayonnement diffusé reçue par le pixel donné n lors de la première acquisition,
- l'intensité $I_2$ du rayonnement lu dans le pixel donné n, pour la deuxième trame d'image : $I_2(n) = q_2(n)P_2(n) + S_2(n)$, avec $q_2$ un facteur prédéterminé propre au pixel n, $P_2$ l'intensité de rayonnement primaire reçue par le pixel n lors de la deuxième acquisition, $S_2$ l'intensité de rayonnement primaire reçue par le pixel n lors de la deuxième acquisition.

Les termes $I_1(n)$ et $I_2(n)$ sont ainsi constitués d'une fraction du rayonnement primaire reçu par le pixel n, ainsi que du rayonnement qui, du fait de la diffusion, atteint le pixel n.

Pour un pixel tel que par exemple le pixel pi sur les figures 4A-4B est directement éclairé lors de l'acquisition de la première trame et se trouve dans l'ombre lors de l'acquisition de la deuxième trame, on peut considérer $q_1=1$ et $q_2=0$.

Pour d'autres pixels pi+1, ..., pj-1 se situant dans la pénombre, on considère $q_1$ et $q_2$ variant dans l'intervalle ]0 ;1[.

Pour un pixel tel que par exemple le pixel pj sur les figures 4A-4B se trouvant dans l'ombre lors de l'acquisition de la première trame et directement éclairé lors de l'acquisition de la deuxième trame, on peut considérer $q_1 = 0$ et $q_2 = 1$.

Pour deux positions de l'élément bloqueur 20 décalées d'un pas de translation égal à d, on peut faire l'approximation suivante : $S_1 \approx S_2$ en partant de l'hypothèse qu'il y a une forte corrélation spatiale dans le terme de rayonnement diffusé.

Les facteurs $q_1(n)$ et $q_2(n)$ sont propres au pixel n et représentent des fractions de rayonnement primaire reçu par le pixel n en tenant compte des pénombres géométrique et physique dans les deux positions de l'élément bloqueur 20.

On peut considérer la relation suivante : $q_1 = 1-q_2$.

Des estimations des facteurs $q_1$ et $q_2$ peuvent avoir été réalisées préalablement pour chaque pixel dès lors que l'on connait la géométrie du système, en particulier la taille du foyer de la source 10 de rayonnement X, la largeur d des zones opaques, la largeur $\Delta$ des pixels, la distance source-détecteur DFI, la distance source-bloqueur DFB, et la position relative de chaque élément, i.e. foyer de la source de rayons X, bloqueur 20 et détecteur 40.

Par exemple, la détermination des facteurs $q_1$ et $q_2$ peuvent être réalisée par simulation numérique d'une exposition du détecteur à un rayonnement X, et ce sans objet cible, pour différentes configurations géométriques, i.e. différentes positions et orientations du bloqueur par rapport au détecteur. Dans une variante, on détermine $q_1$ et $q_2$ par des mesures sans objet cible pour différentes configurations géométriques. Pour chaque image ou trame d'image Im_q1, Im_q2 obtenue, on divise l'image par la valeur maximale mesurée dans un pixel : on normalise ainsi les deux trames d'images Im_q1, Im_q2, si bien que les facteurs $q_1$ et $q_2$ sont ensuite compris entre 0 et 1.

Une estimation de la différence $q_1(n)-q_2(n)$ propre à chaque pixel donné n peut également avoir été réalisée en effectuant dans des conditions semblables à celles décrites précédemment mais sans objet cible : une première mesure d'intensité du pixel donné dans la première position de l'élément bloqueur 20 et une deuxième mesure d'intensité du pixel donné dans la deuxième position de l'élément bloqueur 20, l'estimation du facteur $q_1$-$q_2$ dépendant alors de la différence entre ces deux intensités mesurées.

Après ces mesures préalables, les valeurs du facteur $q_1 - q_2$ peuvent être répertoriées dans un fichier de calibration que l'on peut interroger par la suite lorsque l'on effectue le traitement visant à constituer une image d'un objet cible.

Dans cette phase de constitution d'image, pour permettre d'associer à un pixel n donné le bon facteur $q_1(n) - q_2(n)$ préenregistré, on se sert alors de la détermination de la position du bloqueur 20 par rapport au détecteur 40 que l'on a effectué par analyse d'une ou des trames d'image Im_q1, Im_q2, par exemple de la première trame Im_q1.

On peut distinguer alors deux catégories de pixels : une première catégorie de pixels pour lesquels $q_1$-$q_2 \neq 0$ ou |q1 - q2| > z avec z un seuil prédéterminé par exemple de l'ordre de 0.5 et une deuxième catégorie de pixels pour lesquels $q_1$-$q_2$ atteint un seuil proche de 0 ou égal à 0.

Pour un pixel donné n, lorsque pour ce pixel $q_1(n)$-$q_2(n) \neq 0$, on considère que ce pixel donné n appartient à une première catégorie de pixels et on utilise un premier estimateur $\hat{P}$ du rayonnement primaire tel que :

$$\hat{P}(n) = \frac{|I1(n) - I2(n)|}{|q1(n) - q2(n)|}$$

Pour un pixel m tel que |q1 - q2 | $\leq$ z ou égal à 0 on considère que ce pixel m appartient à une deuxième catégorie de pixels et on utilise un deuxième estimateur $\hat{P}'$ du rayonnement primaire tel que :

$$\hat{P}'(n) = I_1(n) + I_2(n) - (S_1(n) + S_2(n))$$

$\hat{P}'(n) = I_1(n) + I_2(n) - 2\hat{S}_{int}(n)$, avec $\hat{S}_{int}(n)$ une valeur estimée par interpolation du rayonnement diffusé du pixel m à partir d'une valeur de rayonnement diffusé calculé pour ses pixels voisins et pour lesquels $|q_1 - q_2| \neq 0$ ou $> z$.

Pour déterminer $\hat{S}_{int}(n)$, une méthode d'interpolation telle que décrite par exemple dans le document « Survey : Interpolation Methods in Medical Image Processing, IEEE transactions on medical imaging, vol.18, n°11, 1999 peut être employée.

Dans l'exemple des figures 4A-4B, des pixels situés au milieu d'un segment entre le pixel $p_i$ et le pixel $p_j$ ou entre le pixel $p_h$ et le pixel $p_i$ ou entre le pixel $p_j$ et le pixel $p_k$ et dont le niveau de gris varie très peu ou ne varie pas entre les deux positions du bloqueur, sont susceptibles d'appartenir à la deuxième catégorie, c'est-à-dire celle pour laquelle $|q_1 - q_2| \leq z$ ou égal à 0.

Ensuite, après constitution de l'image, un traitement par exemple de type filtrage et visant à réduire le bruit peut être effectué.

Le module de traitement 50 informatique évoqué ci-dessus et permettant de produire une image à partir des trames d'images générées par le dispositif de détection 40, peut être doté d'au moins un processeur, d'au moins un module mémoire et d'au moins un périphérique d'entrée. Le processeur peut être, par exemple, un microprocesseur, un FPGA ou un processeur d'unité centrale, ou un réseau de processeurs.

Le module mémoire peut comprendre, par exemple une mémoire morte ROM, une mémoire programme EPROM, une mémoire vive dynamique DRAM ou tout autre type de mémoire RAM, un élément de stockage magnétique ou optique, des registres ou autres mémoires volatiles et/ou non volatiles.

Des algorithmes sous forme d'instructions peuvent être stockés dans la mémoire programme, et permettent d'effectuer les étapes de traitement numérique décrites plus haut pour constituer une image à partir de trames complémentaires.

Un programme, permettant de mettre en oeuvre le procédé de traitement numérique ou un procédé de calibration peut être résidant ou enregistré sur un support par exemple une carte mémoire SDRAM ou un disque DVD-ROM ou Blu-ray ou un disque dur amovible ou un support magnétique ou une clé USB susceptible d'être lu par le module de traitement 50 informatique.

Le module 50 de traitement informatique peut être également relié à un périphérique tel que par exemple un écran 60 permettant d'afficher l'image radiographique constituée (figure 8).

Le module de traitement informatique 50 peut être relié à un réseau, éventuellement par communication sans fil.

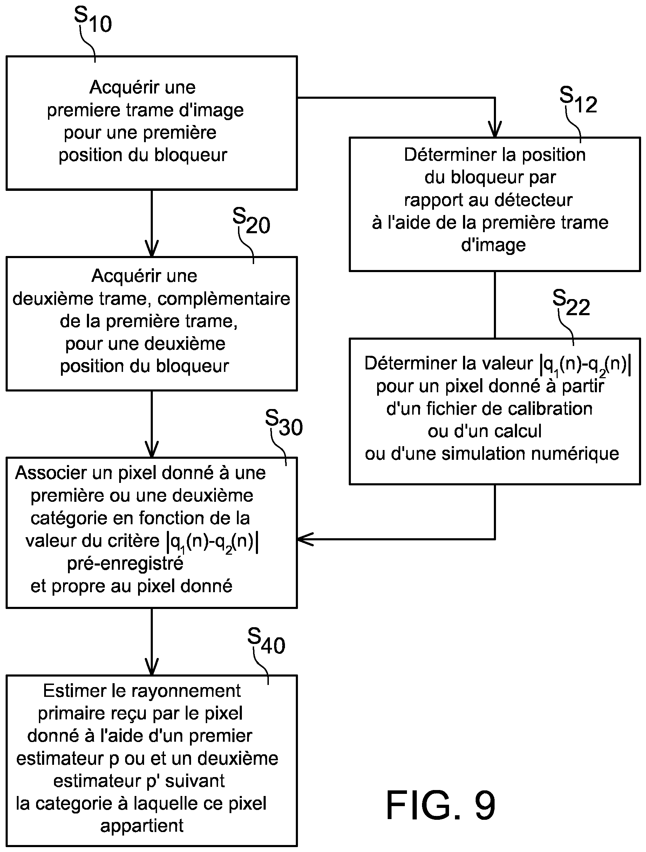

Un exemple d'organigramme reprenant un enchaînement d'étapes de traitement susceptibles d'être effectuées par le module de traitement 50 informatique est donné sur la figure 9.

Une première étape consiste à faire l'acquisition d'une première trame d'image fournie par le détecteur 40 pour la première position de l'élément bloqueur 20 (étape $S_{10}$).

Ensuite, une détermination de la position du bloqueur par rapport au détecteur à l'aide de cette première trame est mise en oeuvre (étape $S_{12}$).

Après la première acquisition, on fait l'acquisition d'une deuxième trame d'image fournie par le détecteur 40 pour la deuxième position de l'élément bloqueur 20 (étape $S_{20}$).

A partir de ces trames d'images complémentaires, on constitue une image en faisant une distinction entre des pixels d'une première catégorie de pixels pour lesquels $q_1 - q_2 \neq 0$ ou bien $q_1 - q_2$ supérieur à un seuil z et des pixels d'une deuxième catégorie d'une deuxième catégorie pour lesquels $q_1 - q_2 = 0$ ou bien $q_1 - q_2$ inférieur à un seuil z (étape $S_{30}$).

Pour cela, on peut avoir, à partir de la connaissance du positionnement du bloqueur 20 par rapport au détecteur 40, avoir déterminé (étape $S_{22}$) la valeur de $q_1 - q_2$, pour chaque pixel à partir de valeurs enregistrées dans un fichier de calibration, les valeurs du fichier de calibration pouvant quant à elles résulter de mesures préalables effectuées dans la première et deuxième position du bloqueur mais sans objet cible.

On estime ensuite (étape $S_{40}$) le rayonnement primaire reçu pour chaque pixel en fonction de la catégorie à laquelle ces pixels appartiennent, un premier estimateur $\hat{P}(n)$ pouvant être utilisé pour les pixels de la première catégorie tandis qu'un deuxième estimateur $P'(n)$ peut être utilisé pour la deuxième catégorie de pixels.

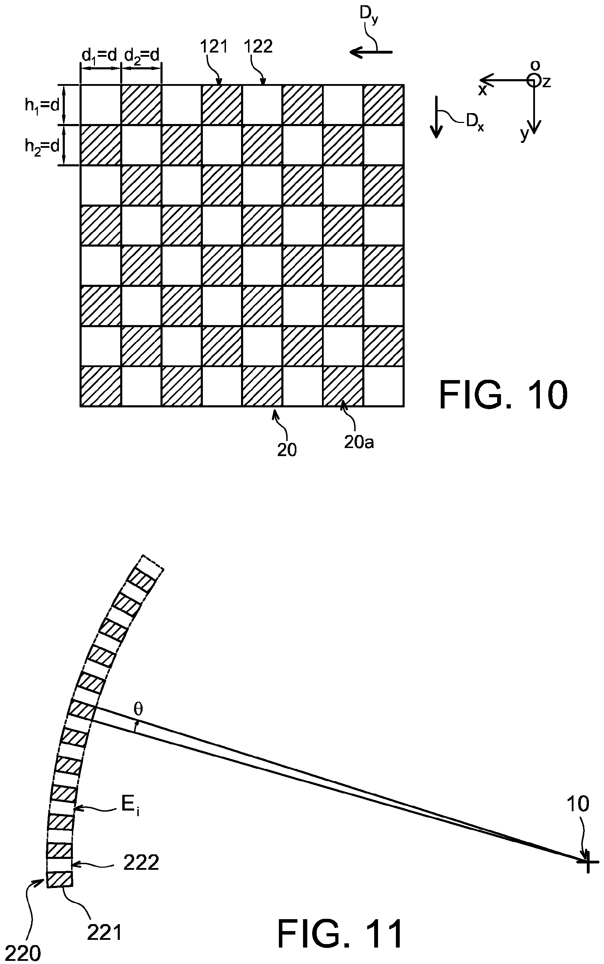

Selon une variante du système d'imagerie précédemment décrit on peut, tel qu'illustré par exemple sur la figure 10, prévoir un élément bloqueur 120 de forme différente et dans lequel des zones opaques 121 et des zones transparentes 122 sont réparties sur deux dimensions selon un agencement en damier. Le bloqueur 120 comprend ainsi plusieurs rangées, chacune comportant une alternance de zones opaques 121 et de zones transparentes de forme d'un carré de côté d.

Le déplacement du bloqueur 120 peut être dans ce cas réalisé par translation selon plusieurs directions Dx, Dy, parallèles aux côtés des carrés et selon un pas $p = k \cdot d$ avec k un entier.

Les exemples de réalisations qui viennent d'être donnés prévoient un mouvement en translation entre les différentes positions d'acquisition d'image.

Un autre mode de réalisation de système imageur illustré sur la figure 11 prévoit un déplacement du bloqueur 220 d'une première position vers une deuxième position complémentaire par rotation, selon un angle de rotation θ par rapport à un axe passant par le foyer de la source 10 de rayons X, θ pouvant être proportionnel à la largeur d'une zone opaque 222.

Pour cette variante de réalisation, le bloqueur 20 peut avoir une section longitudinale incurvée ou un profil incurvé. Dans ce cas, les zones opaques 221 et les zones transparentes 222 de l'élément bloqueur peuvent être de forme rectangulaire ou trapézoïdale et réparties alternativement le long d'une surface incurvée.

Dans l'exemple de la figure 11, des emplacements Ei occupés par les zones transparentes 221 dans une première position du bloqueur 220 sont destinés à être occupés par des zones opaques 222 dans une deuxième position du bloqueur 220 après rotation selon l'angle.

Le profil incurvé est de préférence tel que les zones opaques 221 et transparentes 222 du bloqueur 20 sont réparties sur une portion de sphère et ont avantageusement un agencement en damier.

Avec une telle configuration et un tel mouvement du bloqueur 20, on peut prévoir qu'un emplacement occupé dans la première position par une zone opaque 221 est occupé dans la deuxième position par une zone transparente 222 et inversement, un emplacement occupé dans la première position par une zone transparente 222 est occupé dans la première position par une zone opaque 222. L'amplitude de la rotation pour passer de la première à la deuxième position peut être de l'ordre de la dimension d'une zone opaque 221 ou d'une zone transparente 222.

On peut ainsi obtenir une complémentarité des deux trames d'images obtenues afin de former une image complète et sans artéfact d'un objet examiné.

En variante de l'un ou l'autre des exemples donnés précédemment, on peut également prévoir d'intégrer un bloqueur au sein d'un système d'imagerie dans lequel la source et/ou le détecteur se déplace(nt) par rapport à l'objet dont souhaite acquérir l'image.

Par exemple, un bloqueur tel que décrit précédemment en liaison avec la figure 11 peut être prévu dans un système d'imagerie par tomosynthèse dans lequel la source de rayons X parcourt un arc de cercle tandis que le détecteur reste immobile. Un tel système peut être alors appliqué notamment à la mammographie numérique.

Selon un autre exemple, on prévoit un système d'imagerie avec bloqueur dans lequel la source de rayons X parcourt un arc de cercle tandis que le détecteur réalise également une trajectoire en arc de cercle.

Un autre exemple de réalisation peut prévoir d'intégrer le bloqueur dans un système d'imagerie comportant une source de rayons X destinée à parcourir un arc de cercle tandis que le détecteur se déplace en translation.

Un autre exemple de réalisation peut prévoir d'intégrer le bloqueur au sein d'un système d'imagerie dans lequel la source de rayons X se déplace en translation tandis que le détecteur se déplace également en translation.

Comme cela a été décrit précédemment, le bloqueur déplaçable et formé d'une alternance de zone opaques et de zones transparentes aux rayons X, peut être utilisé pour effectuer une correction du rayonnement diffusé dans une image.

On peut également avoir à déterminer le positionnement du bloqueur par rapport au détecteur afin de connaitre très précisément la distance source détecteur et pouvoir ainsi effectuer une calibration du système d'imagerie.

Une calibration peut s'avérer nécessaire notamment dans un système d'imagerie 3D où la distance source détecteur est susceptible de varier.

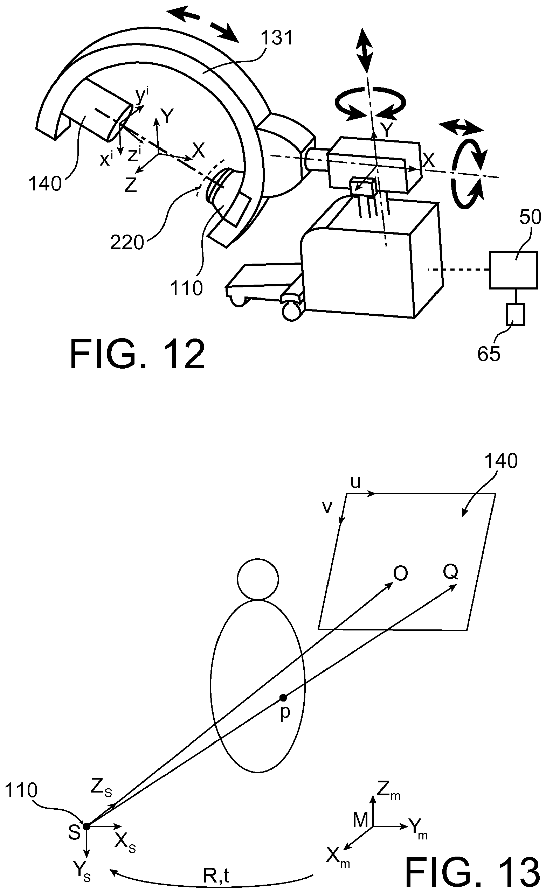

La figure 12 illustre un système d'imagerie par rayons X doté d'un dispositif à arceau (« C-arm » selon la terminologie anglo-saxonne) dans lequel la source 110 de rayons X est liée au détecteur 140 par l'intermédiaire d'un bras 131 en arceau ou en forme de C.

La source 110 et le détecteur 140 sont destinés à tourner autour d'un objet cible, ici un patient, pour réaliser des projections selon plusieurs angles de vue. Ces projections sont ensuite exploitées par l'unité de traitement 50 couplée au détecteur 140.

Dans cet exemple, l'unité de traitement 50 est en outre configurée pour mettre en oeuvre un algorithme de reconstruction afin d'obtenir une image 3D ou volumique, représentant plusieurs coupes de l'objet imagé. Le bloqueur 220 peut être du type de celui décrit précédemment en liaison avec la figure 11 muni de zones opaques et zones transparentes réparties sur une portion de sphère.

Dans ce type de système employé par exemple dans des blocs opératoires, des informations de positionnement relatif de la source 110 et du détecteur 140 sont utilisées afin de construire des images tomographiques 3D. En effet, dans les données d'entrée de l'algorithme de reconstruction utilisé par l'unité de traitement d'image 50 pour produire les images 3D figurent la position du dispositif C-arm relativement à l'objet imagé ainsi que la position relative de la source 110 et du détecteur 140. Or d'éventuelles imperfections mécaniques ainsi que l'effet de la gravité ont tendance à modifier la distance entre la source 110 et le détecteur 140. Il peut être donc prévu d'effectuer une calibration afin de pouvoir connaitre cette information pour chaque projection réalisée.

Il est à noter que, pour effectuer un traitement d'image tel que décrit précédemment en liaison avec la figure 9 et visant à réaliser une correction du diffusé, on peut faire l'acquisition de deux images réelles d'un objet cible dans deux positions différentes du bloqueur. On peut également utiliser deux images de référence $Im\_q_1$, $Im\_q_2$, différentes dans ces deux mêmes positions différentes du bloqueur.

Une image de référence est réalisée sans la présence d'un objet cible entre le bloqueur et le détecteur. Cette image contient donc uniquement des motifs d'éléments du bloqueur.

Pour réaliser cette fois une calibration d'un système d'imagerie par exemple du type de celui illustré sur la figure 12 et connaitre précisément la distance source détecteur, une seule acquisition d'image réelle appelée « image d'alignement » peut être suffisante et on utilise en outre une image de référence Im_$q_1$ ou Im_$q_2$ dans une même position du bloqueur que celle adoptée pour faire l'acquisition de l'image d'alignement.

Afin d'améliorer la précision de calibration, on peut toutefois utiliser au moins une première image de référence Im_$q_1$, et au moins une deuxième image de référence Im_$q_2$ de référence respectivement prises dans une première et une deuxième position du bloqueur 220 et faire l'acquisition d'une premier et une deuxième image d'alignement respectivement dans la première et la deuxième position du bloqueur par rapport à la source.

Ces positions du bloqueur par rapport à la source peuvent être déterminées en usine lors de la fabrication du dispositif, si bien que la distance entre la source 110 et le bloqueur 220 est connue pour chacune de ces positions.

Le déplacement du bloqueur 220 dans ces deux positions est effectué avec une très bonne répétabilité. Etant donné la bonne répétabilité du positionnement du bloqueur 220 par rapport à la source, une calibration pour déterminer la distance source bloqueur ne sera effectuée que peu fréquemment, par exemple lors d'une opération de maintenance du système d'imagerie. La position du bloqueur par rapport à la source est donc considérée comme connue lorsqu'on cherche à déterminer la position du détecteur par rapport à la source.

Le procédé de détermination du positionnement du détecteur 140 par rapport à la source 110, peut comprendre des étapes consistant à :

- disposer le bloqueur 220 entre la source 110 et le détecteur 140,
- générer, une image radiographique dite « d'alignement » comportant des motifs projetés des éléments du bloqueur, en particulier de ses zones opaques et transparentes dans une image radiographique dite d'alignement,
- calculer une position du détecteur 140 à partir des coordonnées des motifs projetés.

En outre, préalablement à l'étape de génération de l'image d'alignement, on peut avoir généré une image radiographique de référence Im_q1 et/ou Im_q2 de manière à pouvoir identifier les transformations géométriques permettant de relier les coordonnées d'éléments du bloqueurs avec les coordonnées de motifs projetés des éléments du bloqueur dans l'image d'alignement.

On se réfère à présent à la figure 13 sur laquelle le système source détecteur est modélisé.

Pour effectuer cette modélisation, l'unité de traitement 50 utilise différents référentiels, dont un référentiel appelé référentiel monde dont le centre est un point M et dans lequel les coordonnées d'un point P sont exprimés par exemple en mètre.

Un référentiel source dont le centre S est la source 110 de rayonnement X et muni d'un premier axe parallèle à un vecteur u, lui-même parallèle au plan image du détecteur 140. Le référentiel source est défini par un deuxième axe parallèle à un autre vecteur v lui-même parallèle au plan image. Un troisième axe du référentiel source est quant à lui normal au plan image. Les coordonnées d'un point exprimé dans le référentiel source peuvent être exprimées par exemple en mètre.

Un référentiel image est également défini à l'aide des vecteurs (u,v) de direction parallèle au détecteur 140, dans lequel l'unité du référentiel est le pixel.

Un point O, appelé « point principal » et qui correspond à une projection orthogonale de l'origine S du référentiel source sur le détecteur 140 est également défini. La distance SO est alors notée f et est appelée la distance focale. Le point O a pour coordonnées (u0,v0) dans le référentiel image, la droite SO étant appelée axe principal.

La projection d'un point P d'un objet sur le plan image est alors un point Q exprimé par une combinaison de transformations géométriques.

Les coordonnées du point P sont exprimées dans le référentiel monde et sont converties dans le référentiel de la source : ce sont des paramètres extrinsèques.

Le point P de l'objet imagé est ensuite projeté sur le plan image (u, v) au point Q en fonction des caractéristiques internes du système source-détecteur : ce sont des paramètres intrinsèques.

Dans le référentiel monde, les coordonnées de P sont : (X,Y,Z)

Dans le référentiel source, les coordonnées de P sont : (Xs,Ys, Zs) On a ainsi la relation :

$$\begin{bmatrix} X_s \\ Y_s \\ Z_s \end{bmatrix} = R \begin{bmatrix} X \\ Y \\ Z \end{bmatrix} + t = [R|t] \begin{bmatrix} X \\ Y \\ Z \end{bmatrix}$$

R, t sont respectivement une opération de rotation et une opération de translation permettant de passer du référentiel monde au référentiel source.

L'opération de rotation R peut être exprimée comme le produit de 3 matrices de rotations, chaque matrice représentant une rotation autour d'un axe du référentiel.

Les coordonnées (x,y,z) dans le référentiel source du projeté Q dans le plan image du point P peuvent être exprimées de la manière suivante à l'aide de la relation de Thalès :

$$\begin{bmatrix} x \\ y \\ z \end{bmatrix} = \begin{bmatrix} f/Z_s & 0 & 0 \\ 0 & f/Z_s & 0 \\ 0 & 0 & f/Z_s \end{bmatrix} \begin{bmatrix} X_s \\ Y_s \\ Z_s \end{bmatrix}$$

Les coordonnées (u,v) du projeté Q dans le référentiel image peuvent être exprimées par la relation suivante :

$$\begin{bmatrix} u \\ v \end{bmatrix} = \begin{bmatrix} k_u & 0 & u_0 \\ 0 & k_v & v_0 \end{bmatrix} \begin{bmatrix} x \\ y \\ 1 \end{bmatrix}$$

avec ku et kv des facteurs de conversion par exemple de mètres en pixels dont la valeur dépend du type de détecteur. Lorsque les pixels du détecteur 140 sont carrés, on a ku=kv=k. Cette expression signifie que le centre du référentiel image (u,v) est dans un coin de l'image et qu'un décalage de u0 et v0 est appliqué pour exprimer les coordonnées par rapport à ce centre.

Par conséquent, on obtient la relation suivante entre Q (u,v) et P(Xs,Ys, Zs) :

$$\begin{bmatrix} au \\ av \\ a \end{bmatrix} = \begin{bmatrix} k \times f & 0 & u_0 \\ 0 & k \times f & v_0 \\ 0 & 0 & 1 \end{bmatrix} \begin{bmatrix} X_s \\ Y_s \\ Z_s \end{bmatrix} = K \begin{bmatrix} X_s \\ Y_s \\ Z_s \end{bmatrix}$$

K est une première matrice de transformation géométrique représentative de paramètres intrinsèques du système source-détecteur : kf, u0 et v0.

Ainsi, la relation entre un point P d'un objet à imager exprimé dans le référentiel monde et son projeté Q dans le plan image est :

$$Q = K \times P$$

Pour déterminer la position du détecteur 140 par rapport à la source 110, l'unité de traitement 50 met en oeuvre une détection de motifs projetés du bloqueur 220 dans au moins une image.

Dans le cas d'un système d'imagerie du type de celui de la figure 12, la distance entre la source 110 et le bloqueur 220 est considérée comme connue. Le référentiel monde est quant à lui placé à la source 110.

L'unité de traitement 50 peut être configurée pour déterminer la position du détecteur 140 à partir d'une position de référence du détecteur 140.

Pour cette position de référence, une matrice de référence Kref avec les paramètres intrinsèques, i.e. f, $u_0$, $v_0$, sont sauvegardés dans une mémoire 55 exploitable par l'unité de traitement 50.

La matrice de référence Kref correspond à une matrice représentative d'une transformation géométrique reliant des coordonnées de motifs projetés d'éléments (zones opaques et/ou transparentes) du bloqueur 220 dans une image de référence Im_q1 ou Im_q2 prise lorsque le système se trouve dans la position de référence avec celles des éléments du bloqueur 220 dans la position de référence.

Le bloqueur 220 est alors à une distance connue du détecteur 140, le détecteur 140 se trouvant à une distance de référence de la source 110. La présence d'un autre objet que le bloqueur 220 n'est pas nécessaire pour faire l'acquisition de cette image de référence. Ainsi l'image de référence Imq1 ou Im_q2 est acquise sans présence d'un objet cible entre le bloqueur et le détecteur.

L'unité de traitement d'image 50 connait ainsi les coordonnées de motifs du bloqueur 220 dans l'image de référence Im_q1 ou Im_q2 prise lorsque le système se trouve dans la position de référence et connait une correspondance entre ces coordonnées et celles des éléments du bloqueur 220 dans le référentiel monde.

Les données relatives à la position de référence peuvent être entrées par le constructeur du système d'imagerie lui-même. Ces données sont alors stockées dans la mémoire 55 couplée à l'unité de traitement 50.

On considère ensuite une position réelle du plan du détecteur 140 que l'on souhaite déterminer. On effectue alors une acquisition d'une image Im_al appelée « image d'alignement ». Cette fois, en plus de la présence du bloqueur 220, un objet cible à étudier que l'on souhaite imager peut être présent entre la source 110 et le détecteur 140.

La position réelle du détecteur 140 est alors exprimée dans un référentiel S2 de la source 110 pour une deuxième vue. Pour cette position réelle, les paramètres intrinsèques sont décrits par une matrice Kali.

Pour faire l'acquisition de l'image réelle, la position du bloqueur 220 par rapport à la source reste inchangée par rapport à celle lors de l'acquisition d'une image de référence correspondante. Autrement dit, dans un cas où le bloqueur 220 se trouve dans la première position par rapport à la source pour faire l'acquisition de l'image réelle, l'image de référence Im_q1 correspondante utilisée pour déterminer la position du détecteur est une image prise lorsque le bloqueur 220 se trouve dans la même première position. Dans un autre cas où le bloqueur 220 se trouve dans la deuxième position pour faire l'acquisition d'une image réelle, l'image de référence Im_q2 correspondante est une image prise lorsque le bloqueur 220 se trouve dans la même deuxième position.

Une opération mathématique reliant les motifs du bloqueur 220 dans la position réelle aux motifs du bloqueur de la position de référence est définie. Cette opération est une homographie planaire. Un tel type d'opération est défini par exemple dans le document « On-line C-arm Intrinsic Calibration: Simulation Study », de B. Spencer & L. Desbat, IEEE Medical imaging conférence 2014. L'unité de de traitement 50 calcule ainsi les paramètres d'une deuxième matrice H de transformation géométrique reliant cette fois des coordonnées de motifs projetés d'éléments (zones opaques et/ou transparentes) du bloqueur 220 dans l'image de référence Im_q1 ou Im_q2 prise lorsque le système se trouve dans la position réelle avec celles des éléments du bloqueur 220 dans la position de référence.

L'unité de traitement 50 peut utiliser une relation générale permettant d'exprimer l'homographie H en fonction d'opérations de rotation et de translation permettant de passer du référentiel source S1 dans la première vue correspondant à l'image de référence au référentiel source S2 dans la deuxième vue correspondant à l'image d'alignement.

Si le référentiel monde est fixé à la source 110, on peut considérer seulement une matrice de rotation R reliant les deux référentiels sources S1 et S2.

Dans ce cas, il n'y a pas de translation, ce qui se traduit par un matrice t de translation telle que t=0. L'Homographie H et la matrice de rotation R sont alors reliées par la formule suivante :

$$HK_{ref} = K_{ali}R$$

Kref est connue et H est calculée par l'unité de traitement 50 à partir des appariements entre les éléments du bloqueur et les motifs projetés.

On peut décomposer HK$_{ref}$ en 4 matrices : une première matrice sous la forme d'une matrice de paramètres intrinsèques qui va représenter la matrice Kali et trois autres matrices qui vont représenter les rotations par rapport aux 3 axes du référentiel S1.

Ces trois matrices représentent la rotation globale qui permet de passer du référentiel S1 au référentiel S2. Ce sont les matrices R et Kali qui renseignent sur la position du détecteur 140 lorsque le système est en cours d'acquisition d'image. L'unité de traitement 50 calcule alors la distance entre source 110 et détecteur 140 à l'aide de ces matrices.

Il peut suffire à l'unité de traitement 50 de 4 points que l'on sait apparier dans les deux positions de référence et réelle pour calculer l'homographie planaire H. Un nombre supérieur de points peut cependant être utilisé.

Un traitement tel que décrit plus haut pour permettre de déterminer les position réelle du détecteur comprend préalablement une détermination de coordonnées de motifs projetés, une détection de motifs projetés du bloqueur 220 dans une image, en particulier dans l'image d'alignement Im_al ou dans l'image de référence Imq1 ou Im_q2, l'unité de traitement 50 peut être configurée pour effectuer une détection de contour. Cette détection est par exemple réalisée au moyen d'opérateurs du type filtre de Canny.

L'unité de traitement 50 peut être également configurée de sorte à détecter des caractéristiques de motifs du bloqueur 220 dans une image, en particulier dans l'image d'alignement ou dans l'image de référence. Des caractéristiques telles que des paramètres géométriques de segments de droites : équation de droite, coordonnées de points extrêmes de segment et de points d'intersection de lignes peuvent être alors obtenues. Une telle détection peut être réalisée par exemple à l'aide d'une transformée de Hough.

Dans le cas, par exemple, où le bloqueur 220 a un agencement de ses zones opaques du type de celui illustré sur la figure 10, une détection d'un motif en damier est mise en oeuvre. Dans un autre cas, par exemple, où le bloqueur est du type de celui illustré sur la figure 2, une détection d'un motif sous forme de bandes parallèles est effectuée. Du fait de l'alternance de zones radio-transparentes et radio-opaques, un motif du bloqueur 220 dans l'image est particulièrement contrasté, ce qui permet de détecter de manière précise les contours des motifs du bloqueur 220 et par voie de conséquence obtenir une finesse accrue de détection de la position du détecteur 140 par rapport à la source 110.

L'unité de traitement 50 d'image est configurée pour apparier des motifs projetés identifiés avec les éléments du bloqueur 220, c'est-à-dire ses zones opaques et/ou transparentes dont l'agencement est connu. Cela peut être réalisé à l'aide d'une table de caractéristiques stockée dans la mémoire 55 couplée à l'unité de traitement 50. Par exemple, la table comprend les caractéristiques des éléments (zones opaques et/ou transparentes) du bloqueur 220 à savoir leur forme, leur longueur et leur position dans le bloqueur 50. Plus particulièrement, l'appariement consiste pour l'unité de traitement 50 à parcourir l'image d'alignement pour identifier des motifs projetés et pour chaque motif projeté identifié, calculer les caractéristiques du motif. Puis l'unité de traitement 50 compare les caractéristiques calculées avec celles de la table et repère l'élément du bloqueur qui correspond au motif projeté.

Pour mettre en correspondance des motifs entre une image de référence Imq1 et/ou Imq2 et une image prise lors d'un examen à l'aide du système tel qu'illustré sur la figure 12, on peut utiliser un critère dit « de faibles déplacements ». En effet, les imprécisions mécaniques du dispositif C-arm sont de l'ordre de quelques mm ou degrés.

On sait donc que le décalage des motifs est faible ce qui permet d'avoir un critère de similarité basé sur la proximité spatiale. Une méthode pour réaliser l'appariement de points de références à des points réels de mesures est par exemple donnée dans le document "Two Dimensional Projective Point Matching", Phd Thesis of Jason Denton, Colorado State University (2002).

L'utilisation d'un bloqueur radio-opaque à plusieurs positions permet d'effectuer une calibration précise du système d'imagerie. Une fois cette calibration effectuée et la distance source détecteur bien déterminée on peut obtenir une image radiographique que l'on peut ensuite corriger à l'aide d'un traitement d'image tel que décrit précédemment en liaison avec la figure 9 et qui permet de corriger la contribution du rayonnement diffusé dans l'image. Le bloqueur 220 ne perturbe pas l'image finale mais permet au contraire de l'améliorer.

**Revendications**

1. Système d'imagerie par rayons X comprenant :

    - une source (10, 110) de rayons X
    - un détecteur (40, 140) de rayons X,
    - un bloqueur (20, 220) formé d'une alternance d'une ou plusieurs zones opaques permettant le blocage de rayons X et d'une ou plusieurs zones transparentes permettant le passage des rayons X,
    - des moyens de déplacement du bloqueur (20, 220) configurés pour déplacer le bloqueur entre au moins une première position et au moins une deuxième position, de sorte que dans la première position, une zone opaque occupe entièrement un premier emplacement donné et une zone transparente occupe entièrement un deuxième emplacement donné, dans la deuxième position, le premier emplacement donné est entièrement occupé par une zone transparente, le deuxième emplacement donné étant entièrement occupé par une zone opaque.
    - des moyens d'acquisition d'image configurés pour réaliser une première acquisition d'une première image lorsque le bloqueur se trouve dans la première position et pour réaliser une deuxième acquisition d'une deuxième image lorsque le bloqueur se trouve dans la deuxième position,
    - une unité de traitement (50) d'image configurée pour déterminer une position du détecteur (40) à partir de coordonnées d'un ou plusieurs motifs projetés du bloqueur (20, 220) dans la première image et/ou dans la deuxième image.

2. Système d'imagerie par rayons X selon la revendication 1, dans lequel l'unité de traitement est en outre configurée pour :

    - déduire d'une position relative du détecteur (40) par rapport au bloqueur (20) l'appartenance d'un pixel donné du détecteur à une première catégorie de pixels ou à une deuxième catégorie de pixels,
    - estimer le rayonnement primaire du pixel donné du détecteur à l'aide d'un premier estimateur $\hat{P}$ lorsque le pixel donné appartient à la première catégorie ou à l'aide d'un deuxième estimateur $\hat{P}'$ lorsque le pixel donné appartient à la deuxième catégorie.

3. Système d'imagerie par rayons X selon la revendication 2, dans lequel l'unité de traitement est en outre configurée pour estimer le rayonnement primaire reçu par un pixel donné n du détecteur à l'aide d'un estimateur $\hat{P}$, dépendant d'un rapport entre :

    - une différence $I_1(n)-I_2(n)$ entre une intensité $I_1(n)$ de rayonnement détectée par le pixel donné n lors de la première acquisition et d'une intensité $I_2(n)$ de rayonnement détectée par le pixel n lors de la deuxième acquisition,
    - une différence $q_1(n)-q_2(n)$, avec $q_1(n)$, $q_2(n)$ étant des paramètres prédéterminés représentatifs de fractions de rayonnement primaire reçus par le pixel n du détecteur dans la première position du bloqueur et dans la deuxième position du bloqueur sans objet cible.

4. Système d'imagerie par rayons X selon la revendication 1 ou 2, dans lequel l'unité de traitement estime le rayonnement primaire reçu par un pixel donné m du détecteur à l'aide d'un estimateur $\hat{P}'$, l'estimation $P'(m)$ du rayonnement primaire reçu par le pixel donné m, étant en fonction d'une intensité $I_1(m)$ de rayonnement primaire détectée par le pixel donné m lors de la première acquisition d'une intensité $I_2(m)$ détectée par le pixel m lors de la deuxième acquisition, et de $S_{int}(m)$ une valeur estimée par interpolation du rayonnement diffusé du pixel n à partir d'une valeur de rayonnement diffusé calculé pour ses pixels voisins.

5. Système d'imagerie selon l'une des revendications 1 à 4, dans lequel le déplacement du bloqueur est une translation du bloqueur (20) d'un pas p donné et dans un plan parallèle au détecteur.

6. Système d'imagerie selon l'une des revendications 1 à 5, dans lequel les zones opaques (21) et les zones transparentes (22) du bloqueur ont une largeur l, le pas p de translation étant égal à $k*l$, avec k un nombre entier.

7. Système d'imagerie selon l'une des revendications 1 à 6, dans lequel l'élément bloqueur (20) comporte une face avant (20a) sur laquelle les zones opaques (21) et les zones transparentes (22) sont réparties sous forme de bandes parallèles ou selon une disposition en damier.

8. Système d'imagerie l'une des revendications 1 à 7, dans lequel les zones opaques (21a) sont dotées de faces longitudinales inclinées les unes par rapport aux autres et suivant des directions qui convergent vers la source de rayons X.

9. Système d'imagerie selon l'une des revendications 1 à 4, dans lequel le déplacement est une rotation du bloqueur (220) autour d'un axe passant par la source de rayons X.

**10.** Système d'imagerie selon la revendication 9, dans lequel les zones opaques et les zones transparentes du bloqueur sont réparties sur une surface incurvée.

**11.** Système d'imagerie selon l'une des revendications 1 à 10, dans lequel l'unité de traitement d'image (50) comporte :

- une mémoire pour stocker des paramètres d'une première matrice de transformation géométrique reliant des coordonnées de motifs de référence avec respectivement les coordonnées d'éléments du bloqueur (20, 220), chaque motif de référence correspondant à une projection d'un élément du bloqueur (20, 220) dans une image radiographique de référence ($Im\_q_1$, $Im\_q_2$) générée lorsque le détecteur est situé à une distance de référence de la source (10, 110) sans objet cible entre le bloqueur et le détecteur, le bloqueur étant alors dans une position donnée parmi lesdites première et deuxième positions,

l'unité de traitement d'image (50) étant en outre configurée pour :

- identifier des motifs projetés d'éléments du bloqueur dans une autre image radiographique dite d'alignement (I1,I2) générée lorsque le bloqueur se trouve dans la position donnée par rapport au détecteur, et pour apparier les motifs projetés dans l'image d'alignement avec respectivement des éléments du bloqueur, pour calculer des paramètres d'une deuxième matrice de transformation géométrique reliant les coordonnées des motifs projetés dans l'image radiographique d'alignement avec les coordonnées des motifs de référence, et pour calculer la position du détecteur à partir des paramètres des premières et deuxième matrices.

**12.** Procédé de calibration d'un système d'imagerie radiographique selon la revendication 11, comprenant les étapes suivantes :

- disposer le bloqueur (20, 200) entre la source (10, 100) et le détecteur (40, 140),
- faire l'acquisition par le détecteur d'au moins une image radiographique d'alignement comportant un ou plusieurs motifs projetés du bloqueur,
- déterminer les coordonnées de motifs projetés du bloqueur dans l'image d'alignement et
- calculer une position du détecteur (40,140) à partir de coordonnées des motifs projetés dans l'image d'alignement.

**Patentansprüche**

**1.** Röntgenbildgebungssystem, umfassend:

- eine Röntgenquelle (10, 110),
- einen Röntgendetektor (40, 140),
- einen Blocker (20, 220), der von einem Wechsel einer oder mehrerer opaker Zonen, die das Blockieren von Röntgenstrahlen ermöglichen, und einer oder mehrerer transparenter Zonen gebildet wird, die den Durchtritt der Röntgenstrahlen ermöglichen,
- Mittel zum Bewegen des Blockers (20, 220), die dafür konfiguriert sind, den Blocker zwischen mindestens einer ersten Stellung und mindestens einer zweiten Stellung zu bewegen, derart, dass in der ersten Stellung eine opake Zone vollständig eine erste gegebene Position einnimmt, und eine transparente Zone vollständig eine zweite gegebene Position einnimmt, in der zweiten Stellung die erste gegebene Position vollständig von einer transparenten Zone eingenommen wird, wobei die zweite gegebene Position vollständig von einer opaken Zone eingenommen wird,
- Bildaufnahmemittel, die dafür konfiguriert sind, eine erste Aufnahme eines ersten Bildes auszuführen, wenn sich der Blocker in der ersten Stellung befindet, und dafür, eine zweite Aufnahme eines zweiten Bildes auszuführen, wenn sich der Blocker in der zweiten Stellung befindet,
- eine Bildverarbeitungseinheit (50), die dafür konfiguriert ist, eine Stellung des Detektors (40) anhand von Koordinaten eines oder mehrerer projizierter Muster des Blockers (20, 220) im ersten Bild und/oder im zweiten Bild zu bestimmen.

**2.** Röntgenbildgebungssystem nach Anspruch 1, wobei die Verarbeitungseinheit weiter dafür konfiguriert ist:

- aus einer relativen Stellung des Detektors (40) in Bezug auf den Blocker (20) die Zugehörigkeit eines gegebenen Pixels des Detektors zu einer ersten Pixelkategorie oder zu einer zweiten Pixelkategorie abzuleiten,
- die Primärstrahlung des gegebenen Pixels des Detektors mithilfe eines ersten Schätzers $\hat{P}$ zu schätzen, wenn das gegebene Pixel zur ersten Kategorie gehört, oder mithilfe eines zweiten Schätzers $\hat{P}'$, wenn das gegebene Pixel zur zweiten Kategorie gehört.

**3.** Röntgenbildgebungssystem nach Anspruch 2, wobei die Verarbeitungseinheit weiter dafür konfiguriert ist, die von einem gegebenen Pixel n des Detektors empfangene Primärstrahlung mithilfe eines Schätzers $\hat{P}$ abhängig von einem Verhältnis zu schätzen

zwischen:

- einer Differenz $I_1(n)-I_2(n)$ zwischen einer von dem gegebenen Pixel n bei der ersten Aufnahme detektierten Strahlungsintensität $I_1(n)$ und einer von dem Pixel n bei der zweiten Aufnahme detektierten Strahlungsintensität $I_2(n)$,
- einer Differenz $q_1(n)-q_2(n)$, wobei $q_1(n)$, $q_2(n)$ vorbestimmte Parameter sind, die für vom Pixel n des Detektors in der ersten Stellung des Blockers und in der zweiten Stellung des Blockers ohne Zielobjekt empfangene Primärstrahlungsfraktionen repräsentativ sind.

4. Röntgenbildgebungssystem nach Anspruch 1 oder 2, wobei die Verarbeitungseinheit die von einem gegebenen Pixel m des Detektors empfangene Primärstrahlung mithilfe eines Schätzers $\hat{P}'$ schätzt, wobei die Schätzung $\hat{P}'(m)$ der vom gegebenen Pixel m empfangenen Primärstrahlung von einer vom gegebenen Pixel m bei der ersten Aufnahme detektierten Primärstrahlungsintensität $I_1(m)$, von einer vom Pixel m bei der zweiten Aufnahme detektierten Intensität $I_2(m)$, und von $\hat{S}$ int(m) abhängig ist, einem durch Interpolation der Streustrahlung des Pixels n anhand eines für seine Nachbarpixel berechneten Streustrahlungswertes geschätzten Wert.

5. Bildgebungssystem nach einem der Ansprüche 1 bis 4, wobei die Bewegung des Blockers eine Translation des Blockers (20) mit einer gegebenen Teilung p und in einer zum Detektor parallelen Ebene ist.

6. Bildgebungssystem nach einem der Ansprüche 1 bis 5, wobei die opaken Zonen (21) und die transparenten Zonen (22) des Blockers eine Breite l aufweisen, wobei die Translationsteilung p gleich k*l ist, wobei k eine ganze Zahl ist.

7. Bildgebungssystem nach einem der Ansprüche 1 bis 6, wobei das Blockerelement (20) eine Vorderseite (20a) umfasst, an der die opaken Zonen (21) und die transparenten Zonen (22) in Form von parallelen Streifen oder gemäß einer schachbrettartigen Anordnung verteilt sind.

8. Bildgebungssystem nach einem der Ansprüche 1 bis 7, wobei die opaken Zonen (21a) mit Längsseiten versehen sind, die in Bezug zueinander und gemäß Richtungen geneigt sind, die zur Röntgenquelle hin konvergieren.

9. Bildgebungssystem nach einem der Ansprüche 1 bis 4, wobei die Bewegung eine Drehung des Blockers (220) um eine durch die Röntgenquelle verlaufende Achse herum ist.

10. Bildgebungssystem nach Anspruch 9, wobei die opaken Zonen und die transparenten Zonen des Blockers auf einer gekrümmten Oberfläche verteilt sind.

11. Bildgebungssystem nach einem der Ansprüche 1 bis 10, wobei die Bildverarbeitungseinheit (50) umfasst:

- einen Speicher, um Parameter einer ersten geometrischen Transformationsmatrix zu speichern, die Koordinaten von Referenzmustern mit jeweils den Koordinaten von Elementen des Blockers (20, 220) verknüpft, wobei jedes Referenzmuster einer Projektion eines Elements des Blockers (20, 220) in einem Referenz-Radiographiebild ($Im\_q_1$, $Im\_q_2$) entspricht, das erzeugt wird, wenn der Detektor in einem Referenzabstand von der Quelle (10, 110) ohne Zielobjekt zwischen dem Blocker und dem Detektor liegt, wobei sich der Blocker dann in einer gegebenen Stellung aus der ersten und zweiten Stellung befindet,

wobei die Bildverarbeitungseinheit (50) weiter dafür konfiguriert ist:

- projizierte Muster von Elementen des Blockers in einem anderen, sogenannten Ausrichtungs-Radiographiebild (I1, I2) zu identifizieren, das erzeugt wird, wenn sich der Blocker in der gegebenen Stellung in Bezug auf den Detektor befindet, und dafür, die projizierten Muster im Ausrichtungsbild mit jeweils Elementen des Blockers zu paaren, dafür, Parameter einer zweiten geometrischen Transformationsmatrix zu berechnen, die die Koordinaten der projizierten Muster im Ausrichtungs-Radiographiebild mit den Koordinaten der Referenzmuster verknüpft, und dafür, die Stellung des Detektors anhand der Parameter der ersten und zweiten Matrix zu berechnen.

12. Verfahren zum Kalibrieren eines Radiographie-Bildgebungssystems nach Anspruch 11, umfassend die folgenden Schritte:

- Anordnen des Blockers (20, 220) zwischen der Quelle (10, 110) und dem Detektor (40, 140),
- Anfertigen der Aufnahme, durch den Detektor, von mindestens einem Ausrichtungs-Radiographiebild, das ein oder mehrere projizierte Muster des Blockers umfasst,
- Bestimmen der Koordinaten von projizierten Mustern des Blockers im Ausrichtungsbild, und
- Berechnen einer Stellung des Detektors (40, 140) anhand von Koordinaten der projizierten Muster im Ausrichtungsbild.

## Claims

1. X-ray imagery system comprising:

   - an X-ray source (10, 110),
   - an X-ray detector (40, 140),
   - a blocker (20, 220) formed from an alternation of one or several opaque zones for blocking X rays and one or several transparent zones that allow X rays to pass,
   - means of displacing the blocker (20, 220) configured to displace the blocker between at least one first position and at least one second position, such that in the first position, an opaque zone entirely occupies a first given position and a transparent zone entirely occupies a second given position, and in the second position, the first given position is entirely occupied by a transparent zone, the second position being entirely occupied by an opaque zone,
   - image acquisition means configured to make a first acquisition of a first image when the blocker is located in the first position and to make a second acquisition of a second image when the blocker is in the second position,
   - an image processing unit (50) configured to determine a position of the detector (40) from the coordinates of one or several projected patterns of the blocker (20, 220) in the first image and in the second image.

2. X-ray imagery system according to claim 1, in which the processing unit is also configured to:

   - deduce whether a given pixel of the detector belongs to a first category of pixels or to a second category of pixels, from a relative position of the detector (40) relative to the blocker (20),
   - estimate the primary radiation of the given pixel of the detector using a first estimator $\hat{P}$ when the given pixel belongs to the first category or using a second estimator $\hat{P}'$ when the given pixel belongs to the second category.

3. X-ray imagery system according to claim 2, in which the processing unit is also configured to estimate the primary radiation received by a given pixel n of the detector using an estimator $\hat{P}$, dependent on a ratio between:

   - a difference $I_1(n)-I_2(n)$ between a radiation intensity $I_1(n)$ detected by the given pixel n during the first acquisition and a radiation intensity $I_2(n)$ detected by the given pixel n during the second acquisition,
   - a difference $q_1(n)-q_2(n)$, in which $q_1(n)$ and $q_2(n)$ are predetermined parameters representative of primary radiation fractions received by pixel n of the detector in the first position of the blocker and in the second position of the blocker without a target object.

4. X-ray imagery system according to claim 1 or 2, in which the processing unit estimates the primary radiation received by a given pixel m of the detector using an estimator $\hat{P}'$, the estimation $\hat{P}'(m)$ of the primary radiation received by the given pixel m being a function of an intensity $I_1(m)$ of primary radiation detected by the given pixel m during the first acquisition of an intensity $I_2(m)$ detected by the pixel m during the second acquisition, and of Sint(m) a value estimated by interpolation of the radiation diffused from pixel n starting from a diffused radiation value calculated for its adjacent pixels.

5. Imagery system according to one of claims 1 to 4, in which the displacement of the blocker is a translation of the blocker (20) by a given pitch p in a plane parallel to the detector.

6. Imagery system according to one of claims 1 to 5, in which the widths of the opaque zones (21) and the transparent zones (22) of the blocker are 1, the translation pitch p being equal to k*l, where k is an integer number.

7. Imagery system according to one of claims 1 to 6, in which the blocker element (20) comprises a front face (20a) on which the opaque zones (21) and the transparent zones (22) are distributed in the form of parallel strips or in a checker board layout.

8. Imagery system according to one of claims 1 to 7, in which the opaque zones (21a) are provided with longitudinal faces inclined relative to each other and along directions that converge towards the X-ray source.

9. Imagery system according to one of claims 1 to 4, in which the displacement is a rotation of the blocker (220) about an axis passing through the X-ray source.

10. Imagery system according to claim 9, in which the opaque zones and the transparent zones are distributed on a curved surface.

11. Imagery system according to one of claims 1 to 10, in which the image treatment unit (50) comprises

    - a memory to store the parameters of a first geometric transformation matrix relating coordinates of reference patterns with respectively coordinates of elements of the blocker (20, 220), each reference pattern corresponding to a projection of an element of the blocker (20, 220) in a reference radiographic image ($Im\_q_1$, $Im\_q_2$) generated when the detector is located at a reference distance from the source (10, 110) without a target object between the blocker and the detector, the blocker then being in a given position among said first and second positions,

the image treatment unit (50) also being configured to:

- identify projected patterns of elements of the blocker in another alignment radiographic image called the alignment image (I1, I2) generated when the blocker is located in the given position relative to the detector, and to match the projected patterns in the alignment image with the corresponding blocker elements, to calculate the parameters of a second geometric transformation matrix relating the coordinates of patterns projected in the radiographic alignment image with the coordinates of reference patterns, and to calculate the position of the detector from the parameters of the first and second matrices.

12. Method of calibrating a radiographic imagery system according to claim 11, comprising the following steps:

- place the blocker (20, 220) between the source (10, 110) and the detector (40, 140),
- make the detector acquire at least one radiographic alignment image comprising one or several projected patterns of the blocker,
- determine the coordinates of projected patterns of the blocker in the alignment image, and
- calculate a position of the detector (40, 140) from the coordinates of patterns projected in the alignment image.

FIG. 1

FIG. 2

FIG. 3

FIG. 4A

# FIG. 4B

FIG. 5A

FIG. 5B

FIG. 6

FIG. 7

FIG. 8

EP 3 172 557 B1

S10

Acquérir une
premiere trame d'image
pour une première
position du bloqueur

S12

Déterminer la position
du bloqueur par
rapport au détecteur
à l'aide de la première trame
d'image

S20

Acquérir une
deuxième trame, complèmentaire
de la première trame,
pour une deuxième
position du bloqueur

S22

Déterminer la valeur $|q_1(n)-q_2(n)|$
pour un pixel donné à partir
d'un fichier de calibration
ou d'un calcul
ou d'une simulation numérique

S30

Associer un pixel donné à une
première ou une deuxième
catégorie en fonction de la
valeur du critère $|q_1(n)-q_2(n)|$
pré-enregistré
et propre au pixel donné

S40

Estimer le rayonnement
primaire reçu par le pixel
donné à l'aide d'un premier
estimateur p ou et un deuxième
estimateur p' suivant
la categorie à laquelle ce pixel
appartient

FIG. 9

FIG. 10

FIG. 11

**FIG. 12**

**FIG. 13**

## RÉFÉRENCES CITÉES DANS LA DESCRIPTION

**Documents brevets cités dans la description**

- FR 2759800
- US 7551716 A
- US 6490475 B
- US 7497621 B

**Littérature non-brevet citée dans la description**

- **SCHÖRNER et al.** Scatter correction method by temporal primary modulation in X-ray CT. *IEEE Transactions on Nuclear Science,* 2012, vol. 59, 3278-3285
- **B. SPENCER ; L. DESBAT.** On-line C-arm Intrinsic Calibration: Simulation Study. *IEEE Medical imaging conférence,* 2014
- **R. SZELISKI.** Computer Vision : algorithms and applications. Springer, 2010
- **R. I. HARTLEY ; A. ZISSERMAN.** Multiple View Geometry in Computer Vision. Cambridge University Press, 2000
- Survey : Interpolation Methods in Medical Image Processing. *IEEE transactions on medical imaging,* 1999, vol. 18 (11
- Two Dimensional Projective Point Matching. Phd Thesis of Jason Denton. Colorado State University, 2002